(19) Europäisches Patentamt
European Patent Office
Office européen des brevets

(11) Veröffentlichungsnummer: **0 003 101**
**A1**

(12) # EUROPÄISCHE PATENTANMELDUNG

(21) Anmeldenummer: 79100007.8

(22) Anmeldetag: 02.01.79

(51) Int. Cl.²: C 07 C 157/14
A 01 N 9/12

(30) Priorität: 13.01.78 DE 2801318

(43) Veröffentlichungstag der Anmeldung: 25.07.79 Patentblatt 79/15

(84) Benannte Vertragsstaaten: BE CH DE FR GB NL

(71) Anmelder: Bayer Aktiengesellschaft
Zentralbereich Patente,Marken und Lizenzen Bayerwerk
D-5090 Leverkusen 1(DE)

(72) Erfinder: Enders, Edgar, Dr.
René-Bohn-Strasse 16
D-5000 Köln 80(DE)

(72) Erfinder: Stendel, Wilhelm, Dr.
In den Birken 55
D-5600 Wuppertal 1(DE)

(72) Erfinder: Hammann, Ingeborg, Dr.
Belfortstrasse 9
D-5000 Köln 1(DE)

(72) Erfinder: Behrenz, Wolfgang, Dr.
Untergründemich 14
D-5063 Overath(DE)

(54) **Substituierte N-Cyclohexyl-isothioharnstoffe, Verfahren zu ihrer Herstellung sowie ihre Verwendung als Mittel zur Bekämpfung von tierischen und pflanzlichen Schädlingen.**

(57) Die vorliegende Erfindung betrifft neue substituierte N-Cyclohexyl-isothioharnstoffe, Verfahren zu ihrer Herstellung sowie ihre Verwendung als Mittel zur Bekämpfung von tierischen und pflanzlichen Schädlingen, insbesondere als Ektoparasitizide, Insektizide und Akarizide.

EP 0 003 101 A1

BAYER AKTIENGESELLSCHAFT 5090 Leverkusen, Bayerwerk
Zentralbereich
Patente, Marken und Lizenzen Er-kl

**Substituierte N-Cyclohexyl-isothioharnstoffe, Verfahren zu ihrer Herstellung sowie ihre Verwendung als Mittel zur Bekämpfung von tierischen und pflanzlichen Schädlingen.**

Die vorliegende Erfindung betrifft neue substituierte N-Cyclohexyl-isothioharnstoffe, Verfahren zu ihrer Herstellung sowie ihre Verwendung als Mittel zur Bekämpfung von tierischen und pflanzlichen Schädlingen, insbesondere als Ektoparasitizide, Insektizide und Akarizide.

Aus der DT-OS 2 337 122 sind N-Aryl-N',N'-dialkyl-thioharnstoffe bekannt, welche gegen Ektoparasiten und insbesondere gegen Zecken wirksam sind.

Demgegenüber zeigen die Verbindungen gemäß vorliegender Anmeldung eine gesteigerte Wirkung gegenüber Zecken (insbesondere gegenüber Phosphorester-resistente Stämme der Gattung boophilus) und zusätzlich eine stark ausgeprägte

Wirksamkeit, welche den Wirkstoffen aus der DT-OS 2 337 122 fehlt.

Vorliegende Erfindung betrifft neue substituierte N-Cyclohexyl-isothioharnstoffe der allgemeinen Formel

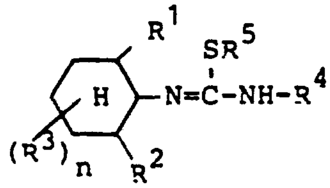

(I)

in welcher

$R^1$ und $R^2$ gleich oder verschieden sein können und für Alkyl oder für gegebenenfalls durch Alkyl substituiertes Cycloalkyl stehen,

$R^3$ für Alkyl oder für gegebenenfalls durch Alkyl substituiertes Cycloalkyl steht,

n für eine ganze Zahl von 0 bis 2,

$R^4$ für Alkyl, Alkenyl oder Cycloalkenyl sowie für gegebenenfalls durch Alkyl, Trifluormethyl oder Cycloalkyl substituiertes Cycloalkyl, und

$R^5$ für Alkyl, Alkenyl oder Cycloalkyl steht.

Die vorliegende Erfindung betrifft insbesondere Verbindungen der Formel

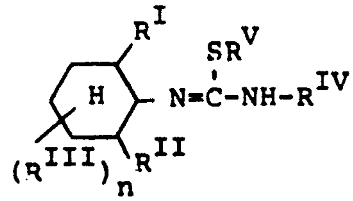

(Ia)

in welcher

$R^I$ für Alkyl ($C_1$-$C_6$) oder Cycloalkyl ($C_5$-$C_6$),

$R^{II}$ für Alkyl ($C_1$-$C_6$) oder Cycloalkyl ($C_5$-$C_6$),

$R^{III}$ für Alkyl ($C_1$-$C_6$) oder Cycloalkyl ($C_5$-$C_6$),

n für eine ganze Zahl von 0 bis 2,

$R^{IV}$ für Alkyl ($C_1$-$C_{12}$); gegebenenfalls durch Alkyl ($C_1$-$C_4$), $CF_3$ oder durch Cycloalkyl ($C_3$-$C_6$) substituiertes Cycloalkyl ($C_3$-$C_{12}$); Alkenyl ($C_2$-$C_{12}$); oder für Cycloalkenyl ($C_5$-$C_6$) und

$R^V$ für Alkyl ($C_1$-$C_6$), Alkenyl ($C_3$-$C_6$) oder Cycloalkyl ($C_3$-$C_6$) stehen.

Die erfindungsgemäßen Verbindungen der Formel (I) zeigen eine stark ausgeprägte Wirksamkeit gegenüber tierischen und pflanzlichen Schädlingen. Sie zeigen insbesondere eine

parasitizide, insektizide und akarizide Wirksamkeit.

Die neuen N-Cyclohexyl-isothioharnstoffe der Formel (I) erhält man, wenn man N-Cyclohexylthioharnstoffe der Formel

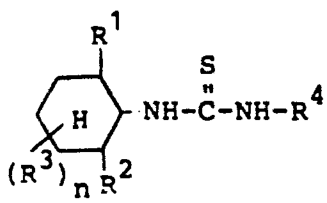

(II)

in welcher $R^1$, $R^2$, $R^3$, $R^4$ und n die oben angegebene Bedeutung besitzen, mit einer Verbindung der allgemeinen Formel

$R^5$-X (III) umsetzt,

in welcher $R^5$ die oben angegebene Bedeutung besitzt und X für einen anionisch abspaltbaren Substituenten steht.

In den Formeln I, II bzw. III haben die Substituenten $R^1$, $R^2$, $R^3$, $R^4$ und $R^5$ vorzugsweise die folgenden Bedeutungen:

Als Alkyl $R^1$, $R^2$ und $R^3$ steht geradkettiges oder verzweigtes Alkyl mit vorzugsweise 1 bis 6, insbesondere 1 bis 4 Kohlenstoffatomen. Beispielhaft seien Methyl, Äthyl, n.- und i.-Propyl, n.-, sek.-, i.- und t.-Butyl, genannt sowie n-Pentyl; Pentyl-(2); Pentyl-(3); tert.-Pentyl; Hexyl-(2); Hexyl-(3).

Alkyl $R^4$ steht für einen geradkettigen oder verzweigten Alkylrest mit vorzugsweise 1 bis 12 und insbesondere 1 bis 10 Kohlenstoffatomen. Beispielhaft seien genannt: Methyl, Äthyl, n.- und i.-Propyl, n.-, sek.-, i.- und t.-Butyl, n-Pentyl, n-Hexyl, n-Octyl, n-Dodecyl, 1-Methyl-propyl, 1-Methyl-butyl, 1,1-Dimethylpropyl.

Als gegebenenfalls durch Alkyl substituiertes Cycloalkyl $R^1$, $R^2$ und $R^3$ steht gegebenenfalls durch Alkyl($C_1$-$C_4$) substituiertes Cycloalkyl mit vorzugsweise 5 bis 6 Kohlenstoffatomen. Beispielhaft seien Cyclopentyl, Cyclohexyl und 1-Methylcyclopentyl genannt.

Als gegebenenfalls durch Alkyl , $CF_3$ oder Cycloalkyl substituiertes Cycloalkyl $R^4$ steht vorzugsweise gegebenenfalls durch vorzugsweise Alkyl ($C_1$-$C_4$), $CF_3$ oder Cycloalkyl ($C_3$-$C_6$) substituiertes Cycloalkyl mit vorzugsweise 3 bis 12, insbesondere 3, 5 oder 6 Kohlenstoffatomen. Beispielhaft seien gegebenenfalls substituiertes Cyclopropyl, 1-Methylcyclopropyl, Cyclobutyl, Cyclopentyl, Cyclohexyl, 2-, 3- oder 4-Trifluormethyl-cyclohexyl, 4-Methyl-3-trifluormethyl-cyclohexyl, 4-Aethyl-cyclohexyl, 2-sek.-Butyl-cyclohexyl, 3,5-Bis-trifluormethyl-cyclohexyl, 2,4-, 2,6-, 2,5- oder 3,5-Dimethylcyclohexyl, Cycloheptyl, Cyclooctyl, Bicyclo[2.2.1]heptyl, Bicyclo[2.2.2]octyl, Adamantyl, Bornyl, Isobornyl, Norbornyl, 2-Methyl-cyclopentyl, 1-Methyl-cyclohexyl, Perhydrobiphenyl, Dekalinyl genannt.

Als Alkenyl $R^4$ steht geradkettiges oder verzweigtes Alkenyl mit vorzugsweise 2 bis 12, insbesondere 2 bis 6 Kohlenstoffatomen. Beispielhaft seien Crotyl, 1-Methyl-allyl, 1,1-Dimethyl-allyl, 3,3-Dimethyl-allyl, 3-Hexyl-allyl genannt.

Als Cycloalkenyl $R^4$ steht vorzugsweise Cycloalkenyl mit 5 bis 6 Kohlenstoffatomen, beispielhaft seien Cyclopenten-(1)-yl-(3), Cyclopenten(1)-yl-(4), Cyclohexen-(1)-yl-(3) genannt.

$R^5$ steht vorzugsweise für Alkyl ($C_1$-$C_6$), Alkenyl ($C_3$-$C_6$) oder Cycloalkyl ($C_3$-$C_6$), beispielsweise für Methyl; Aethyl, Propyl, Isopropyl, n-Butyl, n-Hexyl; Allyl, Crotyl, Cyclopropyl, Cyclopentyl, Cyclohexyl.

Als anionisch abspaltbare Substituenten X der Formel III stehen vorzugsweise folgende Reste: Halogen, insbesondere Chlor, Brom oder Jod; Alkylsulfonyloxy, insbesondere Methylsulfonyloxy, Äthylsulfonyloxy; Arylsulfonyloxy, insbesondere Phenylsulfonyloxy oder X steht für den einwertigen Rest eines Schwefelsäurealkylester, Schwefligsäurealkylester oder Phosphorsäurealkylester wie z.B. $CH_3SO_4$, $C_2H_5SO_4$, $CH_3SO_3$, $C_2H_5SO_3$, $(CH_3)_2PO_4$, $(C_2H_5)_2PO_4$.

Die als Ausgangsprodukte eingesetzten Thioharnstoffe der Formel (II) sind ebenfalls neu, sie können hergestellt werden, indem man

a) Isothiocyanate der Formel

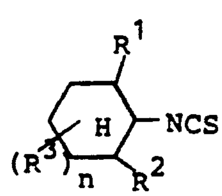

(IV)

in welcher $R^1$, $R^2$, $R^3$ und n die oben angegebene Bedeutung besitzen,

mit Aminen der Formel

$$H_2N\text{-}R^4 \qquad (V)$$

in welcher $R^4$ die oben angegebene Bedeutung besitzt, umsetzt

oder alternativ

b) Isothiocyanate der Formel

$$R^4\text{-}NCS \qquad (VI)$$

in welcher $R^4$ obige Bedeutung besitzt, mit substituierten Cyclohexylaminen der Formel

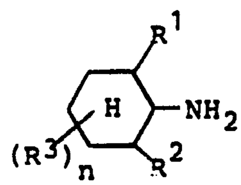

(VII)

in welcher $R^1$, $R^2$, $R^3$ und n die oben angegebene Bedeutung besitzen, umsetzt.

Die für die erfindungsgemäße Umsetzung als Ausgangsverbindungen erforderlichen substituierten Cyclohexylisothiocyanate der allgemeinen Formel IV sind neu. Sie können nach bekannten Methoden aus den entsprechenden Cyclohexylaminen der allgemeinen Formel VII hergestellt werden, beispielsweise durch Umsetzung mit Thiophosgen oder aus den entsprechenden N-Cyclohexyl-dithiocarbonsauren Salzen mit Phosgen, Carbonsäurechloriden oder Oxidationsmitteln (s. Houben Weyl, Methoden der Organischen Chemie, Bd. IX, S. 867-878). Die Cyclohexylaminderivate der allgemeinen Formel VII sind teilweise ebenfalls neu. Sie werden erhalten durch katalytische Hydrierung der entsprechenden 2,6-disubstiutierten Anilinderivate VIIa. Die Cyclohexylaminderivate VII werden dabei als Gemisch der stereomeren Formen erhalten, deren Anteile sich durch gaschromatographische Analyse ermitteln lassen. Die Weiterverarbeitung erfolgt im allgemeinen jeweils in der bei der Hydrierung erhaltenen Zusammensetzung. Demgemäß werden die Isothiocyanate IV, die Thioharnstoffe II und hieraus die erfindungsgemäßen N-Cyclohexyl-isothioharnstoffe I im allgemeinen ebenfalls als Gemische von Stereomeren erhalten. Die Ansprüche der vorliegenden Anmeldung richten sich demgemäß auf alle stereomeren Formen des substituierten Cyclohexanrestes in der allgemeinen Formel I.

Die für die Hydrierung verwendeten 2,6-disubstituierten Anilinderivate VIIa sind ebenfalls teilweise neu. Sie können durch Alkylierung von Anilin und Anilinderivaten mit einer freien Orthostellung zur Aminogruppe nach dem Verfahren von Stroh und Mitarbeitern: Angew. Chemie, Band 69, S. 124 ff. (1957) erhalten werden.

Der Syntheseweg für die erforderlichen Zwischenprodukte kann durch folgendes Schema verdeutlicht werden:

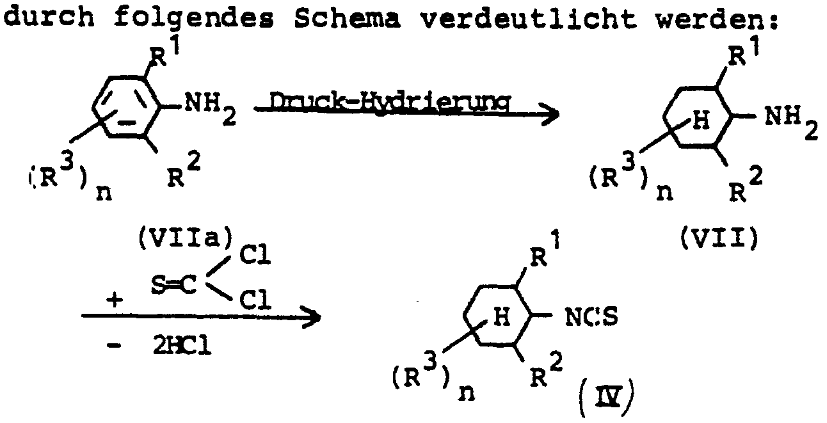


(VIIa) (VII) (IV)

Die Amine der Formel $H_2N-R^4$ (V) sowie die Isothiocyanate der Formel $R^4$-NCS (VI) bekannt oder können nach bekannten Methoden hergestellt werden.
Beispielhaft seien aufgeführt: Methylamin, Äthylamin, Propylamin, Isopropylamin, tert.-Butylamin, Allylamin, Crotylamin, Methyisothiocyanat, Äthylisothiocyanat, Isopropylisothiocyanat, Allylisothiocyanat, Crotylisothiocyanat, Cyclopropylamin, Cyclopentylamin, Cyclohexylisothiocyanat.

Als Beispiele für die erfindungsgemäß als Ausgangsprodukte einsetzbaren Thioharnstoffe der Formel (II) seien aufgeführt:

N-(2,6-Dimethyl-cyclohexyl)-N'-methyl-thioharnstoff
N-(2,6-Dimethyl-cyclohexyl)-N'-alkyl-thioharnstoff
N-(2,6-Dimethyl-cyclohexyl)-N'-propyl-thioharnstoff
N-(2,6-Dimethyl-cyclohexyl)-N'-isopropyl-thioharnstoff
N-(2,6-Dimethyl-cyclohexyl)-N'-n-butyl-thioharnstoff
N-(2,6-Dimethyl-cyclohexyl)-N'-iscbutyl-thioharnstoff
N-(2,6-Dimethyl-cyclohexyl)-N'-sek-butyl-thioharnstoff
N-(2,6-Dimethyl-cyclohexyl)-N'-tert-butyl-thioharnstoff
N-(2,6-Dimethyl-cyclohexyl)-N'-n-pentyl-thioharnstoff
N-(2,6-Dimethyl-cyclohexyl)-N'-pentyl-(2)-thioharnstoff
N-(2,6-Dimethyl-cyclohexyl)-N'-pentyl-(3)-thioharnstoff

N-(2,6-Dimethyl-cyclohexyl)-N'-(3-methyl-butyl)-thioharnstoff
N-(2,6-Dimethyl-cyclohexyl)-N'-[3-methyl-butyl-(2)]-thioharnstoff
N-(2,6-Dimethyl-cyclohexyl)-N'-tert-pentyl-thioharnstoff
N-(2,6-Dimethyl-cyclohexyl)-N'-n-hexyl-thioharnstoff
N-(2,6-Dimethyl-cyclohexyl)-N'-n-dodecyl-thioharnstoff
N-(2,6-Dimethyl-cyclohexyl)-N'-cyclopropyl-thioharnstoff
N-(2,6-Dimethyl-cyclohexyl)-N'-cyclopentyl-thioharnstoff
N-(2,6-Dimethyl-cyclohexyl)-N'-cyclohexyl-thioharnstoff
N-(2,6-Dimethyl-cyclohexyl)-N'-allyl-thioharnstoff
N-(2,6-Dimethyl-cyclohexyl)-N'-methallyl-thioharnstoff
N-(2,6-Dimethyl-cyclohexyl)-N'-buten-(3)-yl-thioharnstoff
N-(2,6-Dimethyl-cyclohexyl)-N'-3,3-dimethyl-allyl-thioharnstoff
N-(2,6-Dimethyl-cyclohexyl)-N'-1-methyl-cyclopentyl-thioharnstoff
N-(2,6-Dimethyl-cyclohexyl)-N'-cyclohexen-(2)-yl-thioharnstoff
N-(2,6-Dimethyl-cyclohexyl)-N'-cyclooctyl-thioharnstoff
N-(2,6-Dimethyl-cyclohexyl)-N'-norbornyl-thioharnstoff
N-(2,6-Dimethyl-cyclohexyl)-N'-bornyl-thioharnstoff
N-(2,6-Dimethyl-cyclohexyl)-N'-adamantyl-thioharnstoff
N-(2-Methyl-6-äthyl-cyclohexyl)-N'-isopropyl-thioharnstoff
N-(2-Methyl-6-äthyl-cyclohexyl)-N'-tert-butyl-thioharnstoff
N-(2-Methyl-6-äthyl-cyclohexyl)-N'-isobutyl-thioharnstoff
N-(2-Methyl-6-äthyl-cyclohexyl)-N'-sek-butyl-thioharnstoff
N-(2-Methyl-6-äthyl-cyclohexyl)-N'-tert-pentyl-thioharnstoff
N-(2-Methyl-6-äthyl-cyclohexyl)-N'-methallyl-thioharnstoff
N-(2-Methyl-6-äthyl-cyclohexyl)-N'-cyclopentyl-thioharnstoff
N-(2-Methyl-6-äthyl-cyclohexyl)-N'-norbornyl-thioharnstoff
N-(2-Methyl-6-äthyl-cyclohexyl)-N'-perhydro-1-naphthyl-thioharnstoff
N-(2-Methyl-6-äthyl-cyclohexyl)-N'-2-methyl-cyclohexyl-thioharnstoff

N-(2,6-Diäthyl-cyclohexyl)-N'-methyl-thioharnstoff
N-(2,6-Diäthyl-cyclohexyl)-N'-äthyl-thioharnstoff
N-(2,6-Diäthyl-cyclohexyl)-N'-isopropyl-thioharnstoff
N-(2,6-Diäthyl-cyclohexyl)-N'-isobutyl-thioharnstoff
N-(2,6-Diäthyl-cyclohexyl)-N'-n-butyl-thioharnstoff
N-(2,6-Diäthyl-cyclohexyl)-N'-tert-butyl-thioharnstoff
N-(2,6-Diäthyl-cyclohexyl)-N'-sek-butyl-thioharnstoff
N-(2,6-Diäthyl-cyclohexyl)-N'-tert-pentyl-thioharnstoff
N-(2,6-Diäthyl-cyclohexyl)-N'-2-pentyl-thioharnstoff
N-(2,6-Diäthyl-cyclohexyl)-N'-norbornyl-thioharnstoff
N-(2,6-Diäthyl-cyclohexyl)-N'-bornyl-thioharnstoff
N-(2-Methyl-6-isopropyl-cyclohexyl)-N'-isopropyl-thioharnstoff
N-(2-Methyl-6-isopropyl-cyclohexyl)-N'-isobutyl-thioharnstoff
N-(2-Methyl-6-isopropyl-cyclohexyl)-N'-tert-butyl-thioharnstoff
N-(2-Methyl-6-isopropyl-cyclohexyl)-N'-tert-pentyl-thioharnstoff
N-(2-Methyl-6-isopropyl-cyclohexyl)-N'-cyclopentyl-thioharnstoff
N-(2,6-Diisopropyl-cyclohexyl)-N'-methyl-thioharnstoff
N-(2,6-Diisopropyl-cyclohexyl)-N'-äthyl-thioharnstoff
N-(2,6-Diisopropyl-cyclohexyl)-N'-isopropyl-thioharnstoff
N-(2,6-Diisopropyl-cyclohexyl)-N'-sek-butyl-thioharnstoff
N-(2,6-Diisopropyl-cyclohexyl)-N'-isobutyl-thioharnstoff
N-(2,6-Diisopropyl-cyclohexyl)-N'-tert-butyl-thioharnstoff
N-(2,6-Diisopropyl-cyclohexyl)-N'-sek-pentyl-thioharnstoff
N-(2,6-Diisopropyl-cyclohexyl)-N'-cyclopropyl-thioharnstoff
N-(2,6-Diisopropyl-cyclohexyl)-N'-cyclopentyl-thioharnstoff
N-(2,6-Diisopropyl-cyclohexyl)-N'-pentyl-(2)-thioharnstoff
N-(2,6-Diisopropyl-cyclohexyl)-N'-hexyl-(3)-thioharnstoff
N-(2,6-Diisopropyl-cyclohexyl)-N'-(2,2-dimethyl-propyl)-thioharnstoff

N-(2,6-Diisopropyl-cyclohexyl)-N'-[2,3-dimethyl-butyl-(2)]-thioharnstoff

N-(2,6-Diisopropyl-cyclohexyl)-N'-octyl-(2)-thioharnstoff

N-(2,6-Diisopropyl-cyclohexyl)-N'-norbornyl-thioharnstoff

N-(2,6-Diisopropyl-cyclohexyl)-N'-bornyl-thioharnstoff

N-(2,6-Diisopropyl-cyclohexyl)-N'-isopropyl-cyclohexyl-thioharnstoff

N-(2-Isopropyl-6-isobutyl-cyclohexyl)-N'-isopropyl-thioharnstoff

N-(2,6-Diisobutyl-cyclohexyl)-N'-isopropyl-thioharnstoff

N-(2,4,6-Trimethyl-cyclohexyl)-N'-isopropyl-thioharnstoff

N-(2,4,6-Trimethyl-cyclohexyl)-N'-tert-butyl-thioharnstoff

N-(2,4,6-Trimethyl-cyclohexyl)-N'-norbornyl-thioharnstoff

N-(4-Methyl-2,6-diäthyl-cyclohexyl)-N'-isopropyl-thioharnstoff

N-(4-Methyl-2,6-diäthyl-cyclohexyl)-N'-tert-butyl-thioharnstoff

N-(4-Methyl-2,6-diäthyl-cyclohexyl)-N'-isobutyl-thioharnstoff

N-(4-Methyl-2,6-diäthyl-cyclohexyl)-N'-sek-butyl-thioharnstoff

N-(4-Methyl-2,6-diäthyl-cyclohexyl)-N'-[2,3-dimethyl-butyl-(1)]-thioharnstoff

N-(4-Methyl-2,6-diäthyl-cyclohexyl)-N'-cyclopentyl-thioharnstoff

N-(4-Methyl-2,6-diäthyl-cyclohexyl)-N'-norbornyl-thioharnstoff

N-(4-Methyl-2,6-diäthyl-cyclohexyl)-N'-perhydronaphthyl-(1)-thioharnstoff

N-(2,4-Dimethyl-6-äthyl-cyclohexyl)-N'-isopropyl-thioharnstoff

N-(2,4-Dimethyl-6-isopropyl-cyclohexyl)-N'-tert-butyl-thioharnstoff

N-(2,4-Dimethyl-6-isopropyl-cyclohexyl)-N'-sek-butyl-thioharnstoff

N-(3-Methyl-2,6-diäthyl-cyclohexyl)-N'-äthyl-thioharnstoff

N-(3-Methyl-2,6-diäthyl-cyclohexyl)-N'-isopropyl-thioharnstoff

N-(3-Methyl-2,6-diäthyl-cyclohexyl)-N'-n-butyl-thioharnstoff

N-(3-Methyl-2,6-diäthyl-cyclohexyl)-N'-isobutyl-thioharnstoff

N-(3-Methyl-2,6-diäthyl-cyclohexyl)-N'-sek-butyl-thioharnstoff

N-(3-Methyl-2,6-diäthyl-cyclohexyl)-N'-tert-butyl-thioharnstoff

N-(3-Methyl-2,6-diäthyl-cyclohexyl)-N'-norbornyl-thioharnstoff

N-(3-Methyl-2,6-diäthyl-cyclohexyl)-N'-methallyl-thioharnstoff

N-(3-Methyl-2,6-diäthyl-cyclohexyl)-N'-perhydronaphthyl-(1)-thioharnstoff

N-(3-Methyl-2,6-diäthyl-cyclohexyl)-N'-perhydroindanyl-(2)-thioharnstoff

N-(2,4,6-Triäthyl-cyclohexyl)-N'-isopropyl-thioharnstoff

N-(2,4,6-Triäthyl-cyclohexyl)-N'-isobutyl-thioharnstoff

N-(2,4,6-Triäthyl-cyclohexyl)-N'-tert-butyl-thioharnstoff

N-(2,4,6-Triäthyl-cyclohexyl)-N'-n-pentyl-thioharnstoff

N-(2,4,6-Triäthyl-cyclohexyl)-N'-[2-methyl-hexyl-(2)]-thioharnstoff

N-(2,4,6-Triäthyl-cyclohexyl)-N'-norbornyl-thioharnstoff

N-(2,4,6-Triäthyl-cyclohexyl)-N'-isopropylcyclohexyl-thioharnstoff

N-(3,4-Dimethyl-2,6-diäthyl-cyclohexyl)-N'-isopropyl-thioharnstoff

N-(3,5-Dimethyl-2,6-diäthyl-cyclohexyl)-N'-tert-butyl-thioharnstoff

N-(4-n-Propyl-2,6-diäthyl-cyclohexyl)-N'-isopropyl-thioharnstoff

N-(4-n-Propyl-2,6-diäthyl-cyclohexyl)-N'-tert-butyl-thioharnstoff

N-(4-n-Propyl-2,6-diäthyl-cyclohexyl)-N'-norbornyl-thioharnstoff

N-(4-Isopropyl-2,6-diäthyl-cyclohexyl)-N'-isopropyl-thioharnstoff

N-(4-Isopropyl-2,6-diäthyl-cyclohexyl)-N'-isopropyl-thioharnstoff

N-(4-Isopropyl-2,6-diäthyl-cyclohexyl)-N'-tert-butyl-thioharnstoff

N-(4-Isopropyl-2,6-diäthyl-cyclohexyl)-N'-sek-butyl-thioharnstoff

N-(4-Isopropyl-2,6-diäthyl-cyclohexyl)-N'-Cyclopentyl-thioharnstoff

N-(4-Isopropyl-2,6-diäthyl-cyclohexyl)-N'-octyl-(2)-thioharnstoff

N-(4-n-Butyl-2,6-diäthyl-cyclohexyl)-N'-äthyl-thioharnstoff

N-(4-n-Butyl-2,6-diäthyl-cyclohexyl)-N'-isopropyl-thioharnstoff

N-(4-n-Butyl-2,6-diäthyl-cyclohexyl)-N'-tert-butyl-thioharnstoff

N-(4-n-Butyl-2,6-diäthyl-cyclohexyl)-N'-norbornyl-thioharnstoff

N-(4-Isobutyl-2,6-diäthyl-cyclohexyl)-N'-isopropyl-thioharnstoff N-(4-sek-Butyl-2,6-diäthyl-cyclohexyl)-N'-tert-butyl-thioharnstoff

N-(4-tert-Butyl-2,6-diäthyl-cyclohexyl)-N'-methyl-thioharnstoff

N-(4-tert-Butyl-2,6-diäthyl-cyclohexyl)-N'-äthyl-thioharnstoff

N-(4-tert-Butyl-2,6-diäthyl-cyclohexyl)-N'-propyl-thioharnstoff

N-(4-tert-Butyl-2,6-diäthyl-cyclohexyl)-N'-isopropyl-thioharnstoff

N-(4-tert-Butyl-2,6-diäthyl-cyclohexyl)-N'-n-butyl-thioharnstoff

N-(4-tert-Butyl-2,6-diäthyl-cyclohexyl)-N'-tert-butyl-thioharnstoff

N-(4-tert-Butyl-2,6-diäthyl-cyclohexyl)-N'-norbornyl-thioharnstoff

N-(4-tert-Butyl-2,6-diäthyl-cyclohexyl)-N'-dodecyl-thioharnstoff

N-(4-Cyclopentyl-2,6-diäthyl-cyclohexyl)-N'-isopropyl-thioharnstoff

N-(4-Cyclohexyl-2,6-diäthyl-cyclohexyl)-N'-methyl-thioharnstoff

N-(4-Cyclohexyl-2,6-diäthyl-cyclohexyl)-N'-isopropyl-thioharnstoff

N-(4-Cyclohexyl-2,6-diäthyl-cyclohexyl)-N'-tert-butyl-thioharnstoff

N-(4-Cyclohexyl-2,6-diäthyl-cyclohexyl)-N'-sek-butyl-thioharnstoff

N-(4-Cyclohexyl-2,6-diäthyl-cyclohexyl)-N'-tert-pentyl-thioharnstoff

N-(4-Cyclohexyl-2,6-diäthyl-cyclohexyl)-N'-cyclohexyl-thioharnstoff

N-(4-Cyclohexyl-2,6-diäthyl-cyclohexyl)-N'-hexen-(2)-yl-thioharnstoff

N-(4-n-Hexyl-2,6-diäthyl-cyclohexyl)-N'-äthyl-thioharnstoff

N-(4-n-Hexyl-2,6-diäthyl-cyclohexyl)-N'-isopropyl-thioharnstoff

N-(4-n-Hexyl-2,6-diäthyl-cyclohexyl)-N'-tert-butyl-thioharnstoff

N-(4-n-Hexyl-2,6-diäthyl-cyclohexyl)-N'-cyclopentyl-thioharnstoff

N-(4-Methyl-2,6-diisopropyl-cyclohexyl)-N'-isopropyl-thioharnstoff

N-(2,6-Dicyclopentyl-cyclohexyl)-N'-tert-butyl-thioharnstoff

N-/4-Pentyl-(3)/-2,6-diäthyl-cyclohexyl)-N'-äthyl-thioharnstoff

Die erfindungsgemäß als Ausgangsprodukte einsetzbaren Verbindungen der Formel (III) sind als Alkylierungs-, Alkenylierungs- bzw. Cycloalkylierungsmittel aufzufassen. Sie sind allgemein bekannt. Beispielsweise seien die folgenden aufgeführt:

Methylchlorid, Methylbromid, Methyljodid, Dimethylsulfat, Diäthylsulfat, Dimethylsulfit, Diisopropylsulfit, Crotylbromid, Cyclopentylchlorid, Methansulfonsäuremethylester, Toluolsulfonsäuremethylester, Trimethylphosphat, Fluorsulfonsäuremethylester, Äthylchlorid, Äthylbromid, Äthyljodid, Triäthyloxonium-fluoborat, Propylbromid, Propyljodid, Butylbromid, Butyljodid, Hexyljodid, Allylbromid, Methallylchlorid, Methallylbromid, Buten-(3)-yl-(1)-bromid, 3-Methylbutylbromid, Isobutylbromid, Cyclopropylbromid, Cyclopentylbromid, Cyclohexyljodid.

Bei der erfindungsgemäßen Umsetzung von Verbindungen II mit Verbindungen III arbeitet man im allgemeinen mit mindestens äquimolaren Mengen an Verbindung III, bezogen auf eingesetzten Thioharnstoff II, zweckmäßig jedoch mit einem Überschuß von 5 - 50 Molprozent, vorzugsweise 5 - 30 Molprozent an Verbindung III. Bei schwierig verlaufender Alkylierung, Alkenylierung bzw. Cycloalkenylierung kann auch ein höherer Überschuß verwendet und die Verbindung III anschließend zurückgewonnen werden. Schließlich ist es auch möglich ohne zugesetztes Lösungsmittel in überschüssiger Verbindung III, welche dann die Funktion des Lösungsmittels übernimmt, zu arbeiten.

Die Umsetzung der Thioharnstoffe II mit den Verbindungen III erfolgt im allgemeinen in Lösungsmitteln bei gewöhnlicher oder erhöhter Temperatur, im allgemeinen bei Temperaturen von ca. 10°C bis ca. 120°C, vorzugsweise von ca. 20°C bis ca. 80°C. Geeignete Lösungsmittel sind beispielsweise: Kohlenwasserstoffe oder Halogenkohlenwasserstoffe wie Hexan, Cyclohexan, Methylcyclohexan, Octan, Methylenchlorid, Chloroform, 1,2-Dichloräthylen oder Aromaten wie Benzol, Toluol, Xylol oder verschiedene polare Lösungsmittel wie Äther und Carbonsäurederivate wie Diäthyläther, Diisopropyläther, Tetrahydrofuran, Dioxan, 1,2-Dimethoxyäthan, Acetonitril, Essigsäureäthylester, Dimethylformamid, N-Methyl-pyrrolidinon, Phosphorsäure-trisdimethylamid, Dimethylsulfoxid, Aceton, Butanon, Methanol, Äthanol, Isopropanol.

Die Konzentration an Thioharnstoff II im verwendeten Lösungsmittel wird zweckmäßigerweise möglichst hoch gehalten, vorzugsweise im Bereich 10 - 60 %.

Bei leicht flüchtigen Verbindungen III wie Methylchlorid oder Äthylchlorid, kann auch im geschlossenen Gefäß unter Druck gearbeitet werden.

Bei dem angegebenen Verfahren fallen die erfindungsgemäßen Isothioharnstoffe der allgemeinen Formel I primär in Form ihrer Salze an und zwar in Abhängigkeit von der jeweils verwendeten Verbindung III beispielsweise als Hydrochlorid,

BAD ORIGINAL



Hydrobromid, Hydrojodid, Methansulfonat, p-Toluolsulfonat, Methosulfat, Sulfat.

Im allgemeinen erfolgt bei der Aufarbeitung eine Neutralisation mit einer wäßrigen Base, beispielsweise Sodalösung, Kaliumcarbonatlösung, verdünnte Natronlauge oder ähnliche, wobei die erfindungsgemäßen Isothioharnstoffe I entweder als Lösung in einem wasserunlöslichen Lösungsmittel oder lösungsmittelfrei als viskose Öle unter der wäßrigen Phase anfallen.

Als Isothioharnstoffe der allgemeinen Formel I seien beispielsweise genannt:

N-(2,6-Dimethyl-cyclohexyl)-N'-methyl-S-methyl-isothioharnstoff
N-(2,6-Dimethyl-cyclohexyl)-N'-methyl-S-äthyl-isothioharnstoff
N-(2,6-Dimethyl-cyclohexyl)-N'-methyl-S-allyl-isothioharnstoff
N-(2,6-Dimethyl-cyclohexyl)-N'-methyl-S-crotyl-isothioharnstoff
N-(2,6-Dimethyl-cyclohexyl)-N'-methyl-S-n-hexyl-isothioharnstoff
N-(2,6-Dimethyl-cyclohexyl)-N'-isopropyl-S-methyl-isothioharnstoff
N-(2,6-Dimethyl-cyclohexyl)-N'-isopropyl-S-crotyl-isothioharnstoff
N-(2,6-Dimethyl-cyclohexyl)-N'-n-propyl-S-methyl-isothioharnstoff
N-(2,6-Dimethyl-cyclohexyl)-N'-n-butyl-S-methyl-isothioharnstoff

N-(2,6-Dimethyl-cyclohexyl)-N'-isobutyl-S-methyl-isothioharnstoff

N-(2,6-Dimethyl-cyclohexyl)-N'-sek-butyl-S-methyl-isothioharnstoff

N-(2,6-Dimethyl-cyclohexyl)-N'-tert-butyl-S-methyl-isothioharnstoff

N-(2,6-Dimethyl-cyclohexyl)-N'-pentyl-(2)-S-methyl-isothioharnstoff

N-(2,6-Dimethyl-cyclohexyl)-N'-pentyl-(3)-S-methyl-isothioharnstoff

N-(2,6-Dimethyl-cyclohexyl)-N'-tert-pentyl-S-methyl-isothioharnstoff

N-(2,6-Dimethyl-cyclohexyl)-N'-octyl-(2)-S-methyl-isothioharnstoff

N-(2,6-Dimethyl-cyclohexyl)-N'-dodecyl-S-methyl-isothioharnstoff

N-(2,6-Dimethyl-cyclohexyl)-N'-allyl-S-methyl-isothioharnstoff

N-(2,6-Dimethyl-cyclohexyl)-N'-methallyl-S-methyl-isothioharnstoff

N-(2,6-Dimethyl-cyclohexyl)-N'-cyclohexyl-S-methyl-isothioharnstoff

N-(2,6-Dimethyl-cyclohexyl)-N'-cyclooctyl-S-methyl-isothioharnstoff

N-(2,6-Dimethyl-cyclohexyl)-N'-norbornyl-S-methyl-isothioharnstoff

N-(2,6-Dimethyl-cyclohexyl)-N'-bornyl-S-methyl-isothioharnstoff

N-(2,6-Dimethyl-cyclohexyl)-N'-adamantyl-S-methyl-isothioharnstoff

N-(2-Methyl-6-äthyl-cyclohexyl)-N'-isopropyl-S-methyl-isothioharnstoff

N-(2-Methyl-6-äthyl-cyclohexyl)-N'-isopropyl-S-allyl-isothioharnstoff

N-(2-Methyl-6-äthyl-cyclohexyl)-N'-tert-butyl-S-methyl-isothioharnstoff

N-(2-Methyl-6-äthyl-cyclohexyl)-N'-sek-butyl-S-methyl-isothioharnstoff

N-(2-Methyl-6-äthyl-cyclohexyl)-N'-tert-pentyl-S-methyl-isothioharnstoff

N-(2,6-Diäthyl-cyclohexyl)-N'-äthyl-S-methyl-isothioharnstoff

N-(2,6-Diäthyl-cyclohexyl)-N'-isopropyl-S-methyl-isothioharnstoff

N-(2,6-Diäthyl-cyclohexyl)-N'-sek-butyl-S-methyl-isothioharnstoff

N-(2,6-Diäthyl-cyclohexyl)-N'-tert-butyl-S-methyl-isothioharnstoff

N-(2,6-Diäthyl-cyclohexyl)-N'-tert-pentyl-S-methyl-isothioharnstoff

N-(2,6-Diäthyl-cyclohexyl)-N'-pentyl-(2)-S-methyl-isothioharnstoff

N-(2,6-Diäthyl-cyclohexyl)-N'-pentyl-(3)-S-methyl-isothioharnstoff

N-(2,6-Diäthyl-cyclohexyl)-N'-methyl-S-(3,3-dimethylallyl)-isothioharnstoff

N-(2,6-Diäthyl-cyclohexyl)-N'-äthyl-S-crotyl-isothioharnstoff

N-(2,6-Diäthyl-cyclohexyl)-N'-isopropyl-S-cyclopropyl-isothioharnstoff

N-(2,6-Diäthyl-cyclohexyl)-N'-cyclopentyl-S-methyl-isothioharnstoff

N-(2,6-Diäthyl-cyclohexyl)-N'-(4-methyl-cyclohexyl)-S-methyl-isothioharnstoff

N-(2,6-Diäthyl-cyclohexyl)-N'-perhydronaphthyl-(1)-S-methyl-isothioharnstoff

N-(2,6-Diäthyl-cyclohexyl)-N'-norbornyl-S-methyl-isothioharnstoff

N-(2,6-Diäthyl-cyclohexyl)-N'-norbornyl-S-allyl-isothioharnstoff

N-(2,6-Diäthyl-cyclohexyl)-N'-allyl-S-allyl-isothioharnstoff

N-(2-Äthyl-6-isopropyl-cyclohexyl)-N'-isopropyl-S-methyl-isothioharnstoff

N-(2-Äthyl-6-isopropyl-cyclohexyl)-N'-isobutyl-S-äthyl-isothioharnstoff

N-(2-Äthyl-6-isopropyl-cyclohexyl)-N'-sek-butyl-S-methyl-isothioharnstoff

N-(2-Äthyl-6-isopropyl-cyclohexyl)-N'-tert-butyl-S-methyl-isothioharnstoff

N-(2-Äthyl-6-isopropyl-cyclohexyl)-N'-tert-pentyl-S-methyl-isothioharnstoff

N-(2-Äthyl-6-isopropyl-cyclohexyl)-N'-methyl-S-crotyl-isothioharnstoff

N-(2-Äthyl-6-isopropyl-cyclohexyl)-N'-methyl-S-(3-methyl-butyl)-isothioharnstoff

N-(2-Äthyl-6-isopropyl-cyclohexyl)-N'-norbornyl-S-methyl-isothioharnstoff

N-(2,6-Diisopropyl-cyclohexyl)-N'-isopropyl-S-methyl-isothioharnstoff

N-(2,6-Diisopropyl-cyclohexyl)-N'-isobutyl-S-methyl-isothioharnstoff

N-(2,6-Diisopropyl-cyclohexyl)-N'-tert-butyl-S-methyl-isothioharnstoff

N-(2,6-Diisopropyl-cyclohexyl)-N'-pentyl-(3)-S-methyl-isothioharnstoff

N-(2,6-Diisopropyl-cyclohexyl)-N'-cyclohexyl-S-methyl-isothioharnstoff

N-(2,6-Diisopropyl-cyclohexyl)-N'-norbornyl-S-methyl-isothioharnstoff

N-(2,6-Diisopropyl-cyclohexyl)-N'-bornyl-S-methyl-isothioharnstoff

N-(2,6-Diisopropyl-cyclohexyl)-N'-(1-methyl-cyclopentyl)-S-äthyl-isothioharnstoff

N-(2,6-Diisopropyl-cyclohexyl)-N'-methyl-S-crotyl-isothioharnstoff

N-(2,6-Diisopropyl-cyclohexyl)-N'-methyl-S-buten-(3)-yl-isothioharnstoff

N-(2,6-Diisopropyl-cyclohexyl)-N'-äthyl-S-(3,3-dimethyl-allyl)-isothioharnstoff

N-(2,6-Diisopropyl-cyclohexyl)-N'-(1,1-dimethyl-allyl)-S-methyl-isothioharnstoff

N-(2,6-Diisopropyl-cyclohexyl)-N'-(cyclohexen-(2)-yl)-S-methyl-isothioharnstoff

N-(2,6-Diisopropyl-cyclohexyl)-N'-äthyl-S-pentyl-isothioharnstoff

N-(2-Isopropyl-6-sek-butyl-cyclohexyl)-N'-isopropyl-S-methyl-isothioharnstoff

N-(2-Isopropyl-6-sek-butyl-cyclohexyl)-N'-butyl-S-methyl-isothioharnstoff

N-(2-Isopropyl-6-sek-butyl-cyclohexyl)-N'-sek-butyl-S-methyl-isothioharnstoff

N-(2-Isopropyl-6-sek-butyl-cyclohexyl)-N'-tert-butyl-S-methyl-isothioharnstoff

N-(2,6-Di-sek-butyl-cyclohexyl)-N'-methyl-S-allyl-isothioharnstoff

N-(2,6-Di-sek-butyl-cyclohexyl)-N'-äthyl-S-äthyl-isothioharnstoff

N-(2,6-Di-sek-butyl-cyclohexyl)-N'-isopropyl-S-methyl-isothioharnstoff

N-(2,6-Di-sek-butyl-cyclohexyl)-N'-tert-butyl-S-methyl-isothioharnstoff

N-(2-Äthyl-6-cyclopentyl-cyclohexyl)-N'-isopropyl-S-methyl-isothioharnstoff

N-(2,6-Dicyclopentyl-cyclohexyl)-N'-tert-butyl-S-methyl-isothioharnstoff

N-(2-Äthyl-6-cyclohexyl-cyclohexyl)-N'-isopropyl-S-methyl-isothioharnstoff

N-(2,4,6-Trimethyl-cyclohexyl)-N'-isopropyl-S-methyl-isothioharnstoff

N-(4-Methyl-2,6-diäthyl-cyclohexyl)-N'-methyl-S-crotyl-isothioharnstoff

N-(4-Methyl-2,6-diäthyl-cyclohexyl)-N'-äthyl-S-allyl-isothioharnstoff

N-(4-Methyl-2,6-diäthyl-cyclohexyl)-N'-isopropyl-S-methyl-isothioharnstoff

N-(4-Methyl-2,6-diäthyl-cyclohexyl)-N'-sek-butyl-S-methyl-isothioharnstoff

N-(4-Methyl-2,6-diäthyl-cyclohexyl)-N'-isobutyl-S-methyl-isothioharnstoff

N-(4-Methyl-2,6-diäthyl-cyclohexyl)-N'-tert-butyl-S-methyl-isothioharnstoff

N-(4-Methyl-2,6-diäthyl-cyclohexyl)-N'-pentyl-(2)-S-methyl-isothioharnstoff

N-(4-Methyl-2,6-diäthyl-cyclohexyl)-N'-tert-pentyl-S-methyl-isothioharnstoff

N-(4-Methyl-2,6-diäthyl-cyclohexyl)-N'-(2,2-dimethyl-propyl)-S-methyl-isothioharnstoff

N-(4-Methyl-2,6-diäthyl-cyclohexyl)-N'-octyl-(2)-S-methyl-isothioharnstoff

N-(4-Methyl-2,6-diäthyl-cyclohexyl)-N'-cyclopropyl-S-crotyl-isothioharnstoff

N-(4-Methyl-2,6-diäthyl-cyclohexyl)-N'-cyclobutyl-S-methyl-isothioharnstoff

N-(4-Methyl-2,6-diäthyl-cyclohexyl)-N'-cyclohexyl-S-methyl-isothioharnstoff

N-(4-Methyl-2,6-diäthyl-cyclohexyl)-N'-cyclooctyl-S-methyl-isothioharnstoff

N-(4-Methyl-2,6-diäthyl-cyclohexyl)-N'-norbornyl-S-methyl-isothioharnstoff

N-(4-Methyl-2,6-diäthyl-cyclohexyl)-N'-perhydronaphthyl-(1)-S-methyl-isothioharnstoff

N-(4-Methyl-2,6-diäthyl-cyclohexyl)-N'-(2-isopropyl-cyclohexyl)-S-methyl-isothioharnstoff

N-(2,4-Dimethyl-6-äthyl-cyclohexyl)-N'-tert-butyl-S-methyl-isothioharnstoff

N-(3-Methyl-2,6-diäthyl-cyclohexyl)-N'-S-crotyl-isothioharnstoff

N-(3-Methyl-2,6-diäthyl-cyclohexyl)-N'-isopropyl-S-methyl-isothioharnstoff

N-(3-Methyl-2,6-diäthyl-cyclohexyl)-N'-tert-butyl-S-methyl-isothioharnstoff

N-(3-Methyl-2,6-diäthyl-cyclohexyl)-N'-tert-butyl-S-crotyl-isothioharnstoff

N-(2,4,6-Triäthyl-cyclohexyl)-N'-methyl-S-methyl-isothioharnstoff

N-(2,4,6-Triäthyl-cyclohexyl)-N'-isopropyl-S-methyl-isothioharnstoff

N-(2,4,6-Triäthyl-cyclohexyl)-N'-sek-butyl-S-methyl-isothioharnstoff

N-(2,4,6-Triäthyl-cyclohexyl)-N'-tert-butyl-S-methyl-isothioharnstoff

N-(2,4,6-Triäthyl-cyclohexyl)-N'-tert-pentyl-S-methyl-isothioharnstoff

N-(2,4,6-Triäthyl-cyclohexyl)-N'-pentyl-(3)-S-methyl-isothioharnstoff

N-(2,4,6-Triäthyl-cyclohexyl)-N'-cyclopropyl-S-crotyl-isothioharnstoff

N-(2,4,6-Triäthyl-cyclohexyl)-N'-cyclohexyl-S-methyl-isothioharnstoff

N-(2,4,6-Triäthyl-cyclohexyl)-N'-dodecahydro-biphenyl-S-methyl-isothioharnstoff

N-(2,4,6-Triäthyl-cyclohexyl)-N'-norbornyl-S-methyl-isothioharnstoff

N-(3,4-Dimethyl-2,6-diäthyl-cyclohexyl)-N'-isopropyl-S-methyl-isothioharnstoff

N-(3,5-Dimethyl-2,6-diäthyl-cyclohexyl)-N'-tert-butyl-S-methyl-isothioharnstoff

N-(4-n-Propyl-2,6-diäthyl-cyclohexyl)-N'-isopropyl-S-methyl-isothioharnstoff

N-(4-n-Propyl-2,6-diäthyl-cyclohexyl)-N'-sek-butyl-S-methyl-isothioharnstoff

N-(4-n-Propyl-2,6-diäthyl-cyclohexyl)-N'-tert-butyl-S-methyl-isothioharnstoff

N-(4-n-Propyl-2,6-diäthyl-cyclohexyl)-N'-methyl-S-crotyl-isothioharnstoff
N-(4-n-Propyl-2,6-diäthyl-cyclohexyl)-N'-cyclopropyl-S-methyl-isothioharnstoff
N-(4-n-Propyl-2,6-diäthyl-cyclohexyl)-N'-norbornyl-S-methyl-isothioharnstoff
N-(4-Isopropyl-2,6-diäthyl-cyclohexyl)-N'-methyl-S-crotyl-isothioharnstoff
N-(4-Isopropyl-2,6-diäthyl-cyclohexyl)-N'-isopropyl-S-methyl-isothioharnstoff
N-(4-Isopropyl-2,6-diäthyl-cyclohexyl)-N'-propyl-S-methyl-isothioharnstoff
N-(4-Isopropyl-2,6-diäthyl-cyclohexyl)-N'-butyl-S-methyl-isothioharnstoff
N-(4-Isopropyl-2,6-diäthyl-cyclohexyl)-N'-tert-butyl-S-methyl-isothioharnstoff
N-(4-Isopropyl-2,6-diäthyl-cyclohexyl)-N'-cyclopentyl-S-methyl-isothioharnstoff
N-(4-Isopropyl-2,6-diäthyl-cyclohexyl)-N'-octyl-(2)-methyl-isothioharnstoff
N-(4-Isopropyl-2,6-diäthyl-cyclohexyl)-N'-dodecyl-S-methyl-isothioharnstoff
N-(4-Isopropyl-2,6-diäthyl-cyclohexyl)-N'-norbornyl-S-methyl-isothioharnstoff
N-(4-n-Butyl-2,6-diäthyl-cyclohexyl)-N'-methyl-S-methyl-isothioharnstoff
N-(4-n-Butyl-2,6-diäthyl-cyclohexyl)-N'-äthyl-S-methyl-isothioharnstoff
N-(4-n-Butyl-2,6-diäthyl-cyclohexyl)-N'-propyl-S-propyl-isothioharnstoff

N-(4-n-Butyl-2,6-diäthyl-cyclohexyl)-N'-isopropyl-S-allyl-isothioharnstoff

N-(4-n-Butyl-2,6-diäthyl-cyclohexyl)-N'-isopropyl-S-methyl-isothioharnstoff

N-(4-n-Butyl-2,6-diäthyl-cyclohexyl)-N'-tert-butyl-S-methyl-isothioharnstoff

N-(4-Isobutyl-2,6-diäthyl-cyclohexyl)-N'-isopropyl-S-methyl-isothioharnstoff

N-(4-Isobutyl-2,6-diäthyl-cyclohexyl)-N'-tert-butyl-S-methyl-isothioharnstoff

N-(4-sek-Butyl-2,6-diäthyl-cyclohexyl)-N'-isopropyl-S-methyl-isothioharnstoff

N-(4-sek-Butyl-2,6-diäthyl-cyclohexyl)-N'-sek-butyl-S-methyl-isothioharnstoff

N-(4-sek-Butyl-2,6-diäthyl-cyclohexyl)-N'-tert-butyl-S-methyl-isothioharnstoff

N-(4-sek-Butyl-2,6-diäthyl-cyclohexyl)-N'-pentyl-(3)-S-methyl-isothioharnstoff

N-(4-tert-Butyl-2,6-diäthyl-cyclohexyl)-N'-isopropyl-S-methyl-isothioharnstoff

N-(4-tert-Butyl-2,6-diäthyl-cyclohexyl)-N'-tert-butyl-S-methyl-isothioharnstoff

N-(4-tert-Butyl-2,6-diäthyl-cyclohexyl)-N'-methyl-S-crotyl-isothioharnstoff

N-(4-tert-Butyl-2,6-diäthyl-cyclohexyl)-N'-pentyl-(2)-S-methyl-isothioharnstoff

N-(4-n-Pentyl-2,6-diäthyl-cyclohexyl)-N'-isopropyl-S-methyl-isothioharnstoff

N-(4-n-Hexyl-2,6-diäthyl-cyclohexyl)-N'-isopropyl-S-methyl-isothioharnstoff

N-(4-Cyclopentyl-2,6-diäthyl-cyclohexyl)-N'-isopropyl-S-methyl-isothioharnstoff

N-(4-Cyclopentyl-2,6-diäthyl-cyclohexyl)-N'-tert-butyl-S-methyl-isothioharnstoff

N-(4-Cyclopentyl-2,6-diäthyl-cyclohexyl)-N'-methyl-S-methyl-isothioharnstoff

N-(4-Cyclohexyl-2,6-diäthyl-cyclohexyl)-N'-methyl-S-methyl-isothioharnstoff

N-(4-Cyclohexyl-2,6-diäthyl-cyclohexyl)-N'-propyl-S-methyl-isothioharnstoff

N-(4-Cyclohexyl-2,6-diäthyl-cyclohexyl)-N'-isopropyl-S-methyl-isothioharnstoff

N-(4-Cyclohexyl-2,6-diäthyl-cyclohexyl)-N'-tert-butyl-S-methyl-isothioharnstoff

N-(4-Cyclohexyl-2,6-diäthyl-cyclohexyl)-N'-tert-pentyl-S-methyl-isothioharnstoff

N-(4-Cyclohexyl-2,6-diäthyl-cyclohexyl)-N'-methyl-S-crotyl-isothioharnstoff

N-(4-Cyclohexyl-2,6-diäthyl-cyclohexyl)-N'-norbornyl-S-methyl-isothioharnstoff

N-(4-Methyl-2,6-diisopropyl-cyclohexyl)-N'-isopropyl-S-methyl-isothioharnstoff

N-[4-Pentyl-(3)-2,6-diäthyl-cyclohexyl]-N'-isopropyl-S-methyl-isothioharnstoff

N-(2-Äthyl-4-methyl-6-sek-butyl-cyclohexyl)-N'-isopropyl-S-methyl-isothioharnstoff

N-(2-Methyl-4,6-di-tert-butyl-cyclohexyl)-N'-isopropyl-S-methyl-isothioharnstoff

N-(2-Äthyl-4-methyl-6-isopropyl-cyclohexyl)-N'-tert-butyl-S-methyl-isothioharnstoff

Die Wirkstoffe eignen sich bei guter Pflanzenverträglichkeit und günstiger Warmblütertoxizität zur Bekämfpung von tierischen Schädlingen, insbesondere Insekten und Spinnentieren, die in der Veterinärmedizin, in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z. B. Oniscus asellus, Armadillidium vulgare, Porcellio scaber.
Aus der Ordnung der Diplopoda z. B. Blaniulus guttulatus.
Aus der Ordnung der Chilopoda z. B. Geophilus carpophagus, Scutigera spec.
Aus der Ordnung der Symphyla z. B. Scutigerella immaculata.
Aus der Ordnung der Thysanura z. B. Lepisma saccharina.
Aus der Ordnung der Collembola z. B. Onychiurus armatus.
Aus der Ordnung der Orthoptera z. B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z. B. Forficula auricularia.
Aus der Ordnung der Isoptera z. B. Reticulitermes spp..
Aus der Ordnung der Anoplura z. B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae,

Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp..

Aus der Ordnung der Lepidoptera z. B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.

Aus der Ordnung der Coleoptera z. B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z. B. Diprion spp., Hoplocampa spp., Lasius spp., Monomorium pharaonis, Vespa spp.

Aus der Ordnung der Diptera z.B. Aëdes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp.,

Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..
Aus der Ordnung der Arachnida z.B. Scorpio maurus, Latrodectus mactans.
Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Die Anwendung der erfindungsgemäßen Wirkstoffe erfolgt in Form ihrer handelsüblichen Formulierungen und/oder den aus diesen Formulierungen bereiteten Anwendungsformen.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gew.-% Wirkstoff, vorzugsweise zwischen 0,01 und 10 Gew.-% liegen.

Die Anwendung geschieht in einer den Anwendungsformen angepaßten üblichen Weise.
Die Anwendung der erfindungsgemäßen Wirkstoffe geschieht im Veterinärsektor in bekannter Weise, wie durch orale Anwendung in Form von beispielsweise Tabletten, Kapseln, Tränken, Granulaten, durch dermale Anwendung in Form beispielsweise des Tauchens (Dippen), Sprühens (Sprayen), Aufgießens (pour-on and spot-on) und des Einpuderns sowie durch parenterale Anwendung in Form beispielsweise der Injektion.

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä. sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwas-

serstoffe sowie Butan, Propan, Stickstoff und Kohlendioxid; als feste Trägerstoffe: natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate: gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehle, Kokosnußschalen, Maiskolben und Tabakstengel; als Emulgier- und/oder schaumerzeugende Mittel: nichtionogene und anionische Emulgatoren, wie Polyoxyäthylen-Fettsäure-Ester, Polyoxyäthylen-Fettalkohol-Äther, z.B. Alkylaryl-polyglykol-äther, Alkylsulfonate, Alkylsulfate, Arylsulfonate sowie Eiweißhydrolysate; als Dispergiermittel: z.B. Lignin, Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Zeckentest

Lösungsmittel: 35 Gewichtsteile Aethylenglykolmonomethylaether
35 Gewichtsteile Nonylphenolpolykolaether

Zwecks Herstellung einer geeigneten Formulierung vermischt man drei Gewichtsteile Wirkstoff mit sieben Teilen des oben angegebenen Lösungsmittel-Emulgator-Gemisches und verdünnt das so erhaltene Emulsionskonzentrat mit Wasser auf die jeweils gewünschte Konzentration.
In diese Wirkstoffzubereitungen werden adulte, vollgesogene Zeckenweibchen der Arten Boophilus microplus (sensibel bzw. resistent) eine Minute lang getaucht. Nach dem Tauchen von je lo weiblichen Exemplaren der verschiedenen Zeckenarten überführt man diese in Petrischalen, deren Boden mit einer entsprechend großen Filterscheibe belegt ist.

Nach lo Tagen wird die Wirksamkeit der Wirkstoffzubereitung bestimmt durch Ermittlung der Hemmung der Eiablage gegenüber unbehandelten Kontrollzecken. Die Wirkung drückt man in Prozent aus, wobei loo % bedeutet, daß keine Eier mehr abgelegt wurden und 0 % besagt, daß die Zecken Eier in normaler Menge ablegten.

Beim oben beschriebenen Zeckentest zeigten unter anderem die Verbindungen aus den Beispielen 2 b, 4, 5, 9, 10, 11, 12, 13, 14, 16, 22, 23, 24, 34, 35, 36, 37, 38 und 39 eine gute Wirksamkeit.

**Test mit parasitierenden Fliegenlarven**

Lösungsmittel: 35 Gew.-Teile Aethylenpolyglykolmonomethyläther
35 Gew.-Teile Nonylphenolpolyglykoläther

Zur Herstellung einer zweckmäßigen Wirkstoffzubereitung vermischt man 30 Gew.-Teile der betreffenden aktiven Substanz mit der angegebenen Menge Lösungsmittel, das den oben genannten Anteil Emulgator enthält und verdünnt das so erhaltene Konzentrat mit Wasser auf die gewünschte Konzentration.

Etwa 20 Fliegenlarven (Lucilia cuprina res.) werden in ein Teströhrchen gebracht, welches ca. 2 $cm^3$ Pferdemuskulatur enthält. Auf dieses Pferdefleisch werden 0,5 ml der Wirkstoffzubereitung gebracht. Nach 24 Stunden wird der Abtötungsgrad in % bestimmt. Dabei bedeuten 100 %, daß alle, und 0 %, daß keine Larven abgetötet worden sind.

Beim oben beschriebenen Test mit parasitierenden Fliegenlarven zeigten unter anderem die Verbindungen aus den Beispielen 1 b, 4, 5, 6, 7, 8, 13, 15, 20, 22, 26, 27, 32 und 33 eine gute Wirksamkeit.

Tetranychus-Test (resistent)

| | | |
|---|---|---|
| Lösungsmittel: | 3 Gewichtsteile | Dimethylformamid |
| Emulgator: | 1 Gewichtsteil | Alkylarylpolyglykoläther |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Mit der Wirkstoffzubereitung werden Bohnenpflanzen (Phaseolus vulgaris), die stark von allen Entwicklungsstadien der gemeinen Spinnmilbe oder Bohnenspinnmilbe (Tetranychus urticae) befallen sind, tropfnaß besprüht.

Nach den angegebenen Zeiten wird die Abtötung in % bestimmt. Dabei bedeutet 100 %, daß alle Spinnmilben abgetötet wurden; 0 % bedeutet, daß keine Spinnmilben abgetötet wurden.

Beim oben beschriebenen Tetranychus-Test zeigten unter anderem die Verbindungen aus den Beispielen 21, 22 und 24 eine gute Wirksamkeit.

Plutella-Test

Lösungsmittel: 3 Gewichtsteile Dimethylformamid
Emulgator : 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Mit der Wirkstoffzubereitung besprüht man Kohlblätter (Brassica oleracea) taufeucht und besetzt sie mit Raupen der Kohlschabe (Plutella maculipennis).

Nach den angegebenen Zeiten wird die Abtötung in % bestimmt. Dabei bedeutet 100 %, daß alle Raupen abgetötet wurden; 0 % bedeutet, daß keine Raupen abgetötet wurden.

Beim oben beschriebenen Plutella-Test zeigten unter anderem die Verbindungen aus den Beispielen 1 b, 2 b, 4, 5, 7, 8, 9, 10, 12, 15, 17, 19, 20, 21, 22, 23, 24, 26, 27, 28, 29, 30 und 31 eine gute Wirksamkeit.

## $LT_{100}$-Test für Dipteren

Testtiere: Aedès aegypti
Zahl der Testtiere:
Lösungsmittel:

2 Gewichtsteile Wirkstoff werden in 1000 Volumenteilen Lösungsmittel aufgenommen. Die so erhaltene Lösung wird mit weiterem Lösungsmittel auf die gewünschten geringeren Konzentrationen verdünnt.

2,5 ml Wirkstofflösung werden in eine Petrischale pipettiert. Auf dem Boden der Petrischale befindet sich ein Filterpapier mit einem Durchmesser von etwa 9,5 cm. Die Petrischale bleibt so lange offen stehen, bis das Lösungsmittel vollständig verdunstet ist. Je nach Konzentration der Wirkstofflösung ist die Menge Wirkstoff pro $m^2$ Filterpapier verschieden hoch. Anschließend gibt man die angegebene Anzahl der Testtiere in die Petrischale und bedeckt sie mit einem Glasdeckel.

Der Zustand der Testtiere wird laufend kontrolliert. Es wird diejenige Zeit ermittelt, welche für einen 100 %igen knock down-Effekt notwendig ist.

Bei diesem Test zeigten unter anderem die Verbindungen aus den Beispielen 1 b, 3 b, 4, 11, 13, 14, 15, 16, 20, 21, 23, 25, 26 und 29 eine gute Wirksamkeit.

## Herstellungsbeispiele

### Beispiel 1

a) Synthese des Thioharnstoff-Ausgangsmaterials

N-(4-Methyl-2,6-diäthyl-cyclohexyl)-N'-isopropyl-thioharnstoff

25 g 4-Methyl-2,6-diäthyl-cyclohexyl-isothiocyanat werden in 40 g einer 70 %igen wäßrigen Lösung von Isopropylamin eingetragen. Die zweiphasige Emulsion wird unter exothermer Reaktion homogen, beim Erkalten tritt wieder Trennung in zwei Phasen ein. Nach 10 Stunden wird der Ansatz mit verdünnter Salzsäure verrührt, das ausgeschiedene Öl in Methylenchlorid aufgenommen, die Lösung mit Wasser gewaschen, über $K_2CO_3$ getrocknet, eingeengt und bei $60^{o}C/1,0$ Torr von Resten Lösungsmittel befreit. Ausbeute 28 g viskoses Öl.

Elementaranalyse, H-NMR- und Massenspektrum stehen mit der angenommenen Konstitution in Übereinstimmung.

Die übrigen, im Anmeldungstext aufgeführten und als Ausgangsprodukte eingesetzten Thioharnstoffe der Formel II können in Analogie zu Beispiel 1 a) aus den entsprechenden Isothiocyanaten und Aminen hergestellt werden.

b) Synthese des Isothioharnstoff-Endprodukts

N-(4-Methyl-2,6-diäthyl-cyclohexyl)-N'-isopropyl-S-methyl-isothioharnstoff

15 g N-(4-Methyl-2,6-diäthyl-cyclohexyl)-N'-isopropylthioharnstoff werden bei 60°C in 15 ml Dimethylformamid gelöst und 9,5 g Methyljodid zugegeben. Nach 16-stündigem Stehen bei 20°C wird der Ansatz mit 150 ml Methylenchlorid, 150 ml 15 %iger Sodalösung und 300 ml Wasser geschüttelt; die Methylenchloridphase wird dann mit Wasser gewaschen, über Kaliumcarbonat getrocknet, eingeengt und bei 60°C/1,0 Torr völlig vom Lösungsmittel befreit.

Ausbeute: 14 g viskoses Öl.
Analyse $C_{16}H_{32}N_2S$;Molg. 284,50

| | | | | |
|---|---|---|---|---|
| ber. | C 67,5 | H 11,3 | N 9,9 | S 11,3 |
| gef. | C 67,7 | H 11,6 | N 9,8 | S 10,8 |
| | C 67,8 | | N 10,0 | S 10,8 |

Das H-NMR- und Massenspektrum stehen mit der angenommenen Konstitution in Übereinstimmung. Verwendet man statt Methyljodid in obigem Beispiel die äquivalente Menge Methylbromid oder Dimethylsulfat, so erhält man die gleiche Verbindung.

Analog können die übrigen in dieser Anmeldung genannten Isothioharnstoffe hergestellt werden.

NMR-, IR- und MS-Spektraldaten von N-(4-Methyl-2,6-diäthyl-cyclohexyl)-N'-isopropyl-S-methyl-isothioharnstoff:

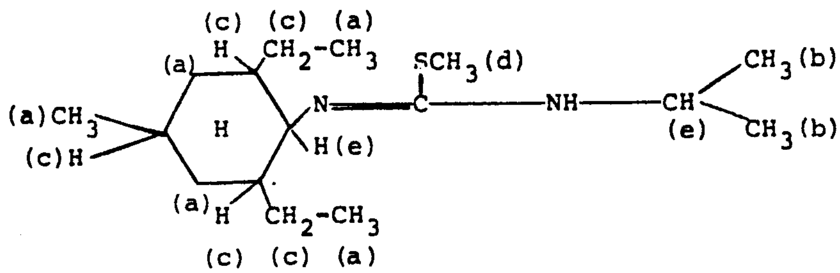

| Shift ppm | Multiplizität | Zuordnung | Intensität |
|---|---|---|---|
| **NMR:** | | | |
| 0,6 - 1,1 | M | I a | 27 |
| 1,14 | C | b | |
| 1,0 - 2,0 | M | c | |
| 2,37 | S | d | 3 |
| 3,4 - 4,1 | M | e | ca. 2 |

<u>IR:</u>

1625 $cm^{-1}$ = C=N-Bande

3430 $cm^{-1}$ = NH-Bande

<u>MS:</u>

Molmasse = 284

Beispiel 2

a) Synthese des Thioharnstoffausgangsmaterials:

N-(2,4,6-Triäthyl-cyclohexyl)-N'-methyl-thioharnstoff

80 g 2,4,6-Triäthyl-cyclohexylamin werden mit 80 ml Aceton verdünnt und 35 g Methylisothiocyanat zugegeben. Die Reaktion verläuft exotherm; man kühlt mit Eiswasser. Nach 10 Stunden verrührt man mit Wasser und verdünnter Salzsäure. Das ausgeschiedene Öl wird mit Methylenchlorid aufgenommen, der Extrakt mit Wasser gewaschen, über Kaliumcarbonat getrocknet, eingeengt und bei 60°C/1 Torr völlig vom Lösungsmittel befreit. Ausbeute 103 g hochviskoses Öl.

Elementaranalyse, HNMR- und Massenspektrum entsprechen der angenommenen Konstitution.

Analog erhält man aus 2,6-Diisopropylcyclohexylamin und Methylisothiocyanat den N-(2,6-Diisopropyl-cyclohexyl)-N'-methyl-thioharnstoff als Feststoff mit einem Schmelzpunktintervall von 110-167°C.

b) Synthese des Isothioharnstoff-Endproduktes:

N-(2,4,6-Triäthyl-cyclohexyl)-N'-methyl-S-crotyl-isothioharnstoff

20 g N-(2,4,6-Triäthyl-cyclohexyl)-N'-methyl-thioharnstoff werden bei 60°C in 20 ml Dimethylformamid gelöst und 11,6 g Crotylbromid zugegeben. Nach 10 Stunden bei

20°C wird der Ansatz mit 150 ml Methylenchlorid, 150 ml 15 %iger Sodalösung und 300 ml Wasser geschüttelt, die Methylenchloridphase mit Wasser gewaschen, über Kaliumcarbonat getrocknet, eingeengt und bei 60°C/1,0 Torr völlig vom Lösungsmittel befreit.

Ausbeute 22 g viskoses Öl.

Elementaranalyse, H-NMR- und Massenspektrum stehen mit der angenommenen Konstitution in Übereinstimmung.

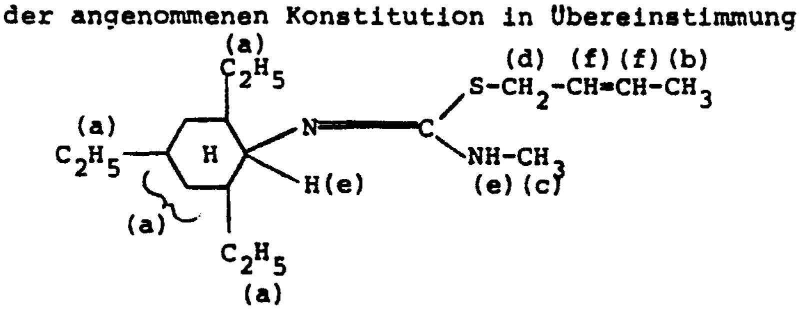

| Shift ppm | Multiplizität | Zuordnung | Intensität |
|---|---|---|---|
| NMR: | | | |
| 0,7 - 2,2 | M | I a | 25 (a + b) |
| 1,75 | D | b | |
| 2,97/2,99 | S/aufgesp. | c | 3 |
| 3,48 | D | d | 2 |
| 3,6 - 4,5 | M | e | ca.2 |
| 5,5 - 5,75 | M | f | 2 |

MS:

Molmasse = 310

Beispiel 3

a) Synthese des Thioharnstoff-Ausgangsmaterials:

N-(2,6-Diäthyl-cyclohexyl)-N'-tert-butyl-thioharnstoff

25 g 2,6-Diäthyl-cyclohexyl-isothiocyanat werden in 30 g tert-Butylamin eingetragen. Die Umsetzung verläuft schwach exotherm. Nach 16 Stunden verrührt man mit verdünnter Salzsäure, verreibt den ausgefallenen Feststoff mit Methanol/Wasser, saugt ab, wäscht und trocknet. Ausbeute 34 g.
Schmelzpunktbereich des Stereomerengemischs 106 - 125°C.
Analyse: $C_{15}H_{30}N_2S$; Molg. 270,47

| | | | | |
|---|---|---|---|---|
| ber. | C 66,6 | H 11,2 | N 10,4 | S 11,9 |
| gef. | C 66,0 | H 12,4 | N 10,4 | S 11,8 |
| | C 66,1 | | N 10,4 | S 12,1 |

Das H-NMR-Spektrum und Massenspektrum stehen mit der angenommenen Konstitution in Übereinstimmung.

b) Synthese des Isothioharnstoff-Endprodukts:

N-(2,6-Diätyhl-cyclohexyl)-N'-tert-butyl-S-methyl-isothioharnstoff

20 g N-(2,6-Diäthyl-cyclohexyl)-N'-tert-butyl-thioharnstoff (Stereomerengemisch) werden bei 60°C in 20 ml Dimethylformamid gelöst und 13 g Methyljodid zugegeben. Nach 10 Stunden bei 20°C wird mit 150 ml Methylenchlorid,

150 ml 15 %iger Sodalösung und 300 ml Wasser geschüttelt. Die Methylenchloridphase wird mit Wasser gewaschen, über Kaliumcarbonat getrocknet, eingeengt und bei 60°C/1,0 Torr völlig vom Lösungsmittel befreit. Ausbeute 19 g viskoses Öl.

Analyse $C_{16}H_{32}N_2S$; Molg. 284,50

| | | | | |
|---|---|---|---|---|
| ber. | C 67,5 | H 11,3 | H 9,9 | S 11,3 |
| gef. | C 68,1 | H 11,5 | H 9,9 | S 11,1 |
| | | | H 10,2 | S 11,2 |

Das H-NMR-Spektrum und das Massenspektrum stehen mit der angenommenen Konstitution in Übereinstimmung.

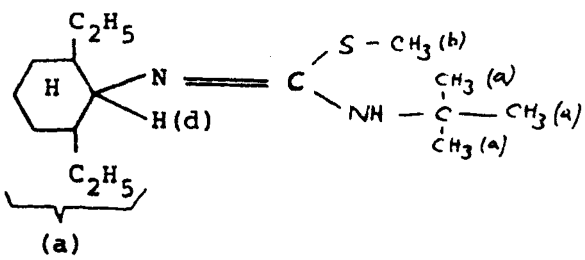

| Shift ppm | Multiplizität | Zuordnung | Intensität |
|---|---|---|---|
| 0,9 - 2,0 | M | I a | 25 |
| 2,3 | S | b | 3 |
| 3,4 | S | c | 1 |
| 3,7 | D | d | 1 |

MS: Molmasse = 284

In Analogie zum Beispiel 1 b) und 2 b) wurden auch die weiteren im folgenden aufgeführten erfindungsgemäßen Isothioharnstoffe dargestellt, wobei unter den Formelbildern wiederum charakteristische NMR-Signale, gegebenenfalls Infrarot (IR)-Banden und Massenspektraldaten (MS) angegebenen sind.

Die Multiplizität der H-NMR-Signale wird jeweils wie folgt abgekürzt:

S = Singulett
D = Dublett
T = Terzett
Q = Quartett
M = Multiplett

Bei den Spektralwerten der Massenspektren (MS) wird die jeweils gefundene Molmasse bzw. Massen von Molekülfragmenten angegeben. Zum Teil werden auch noch charakteristische Banden aus den Infrarot (IR)Spektren angegeben.

Beispiel Nr. 4

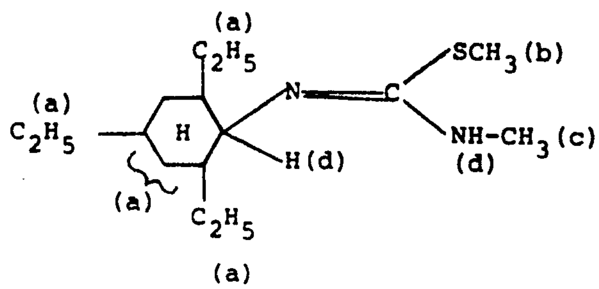

| Shift ppm | Multiplizität | Zuordnung | Intensität |
|---|---|---|---|
| **NMR** | | | |
| 0,7 - 2,2 | M | I a | 22 |
| 2,37 | S (aufgesp.) | b | 3 |
| 2,8 - 3,0 | M | c | ca. 3 |
| 3,6 - 4,4 | M | d | ca. 2 |

MS

Molmasse = 270 (Nebenkomponenten 284, 296, 343)

Beispiel Nr. 5

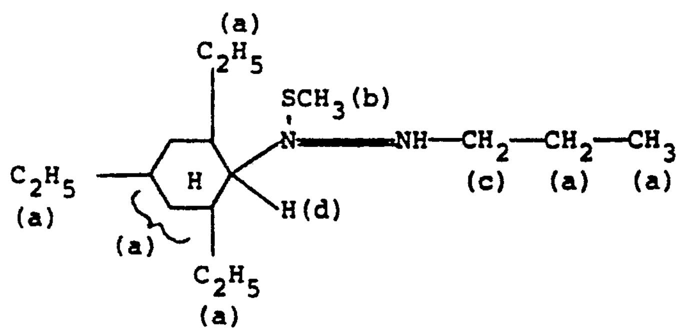

| Shift ppm | Multiplizität | Zuordnung | Intensität |
|---|---|---|---|
| NMR: | | | |
| 0,7 - 2,1 | M | I a | 28 |
| 2,35 (aufgesp.) | S | b | 3 |
| 3,0 - 3,4 | M | c | 2 |
| 4,1 - 4,4 | M | d | 1 |

MS:
Molmasse = 289

Beispiel Nr. 6

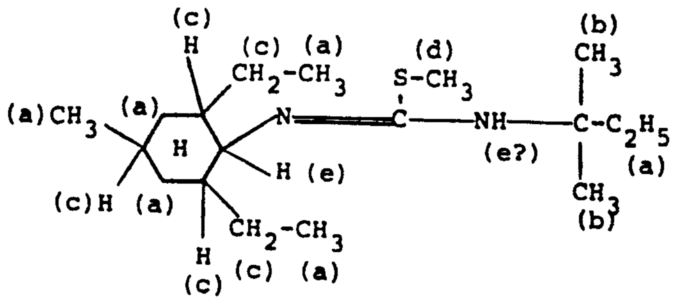

| Shift ppm | Multiplizität | Zuordnung | Intensität |
|---|---|---|---|
| **NMR:** | | | |
| 0,6 - 1,2 | M | I a | |
| 1,28 | S | b | > 31 |
| 1,0 - 2,1 | M | c | |
| 2,34 | S | d | 3 |
| 3,4 - 3,8 | M | e | ca. 2 |

**IR:**

1633 $cm^{-1}$ = C=N-Bande

3440 $cm^{-1}$ = NH-Bande

**MS:**

Molmasse = 312

Beispiel Nr. 7

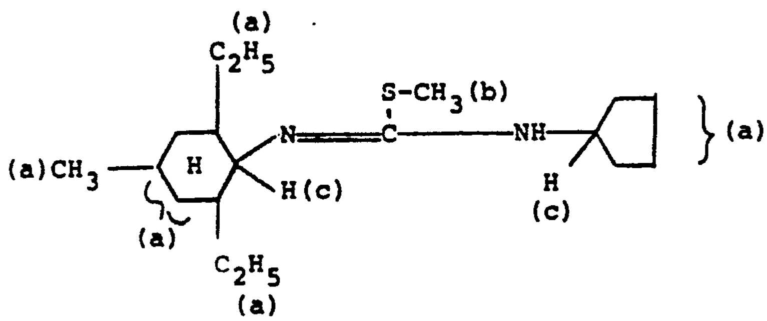

| Shift ppm | Multiplizität | Zuordnung | Intensität |
|---|---|---|---|
| NMR: | | | |
| 0,5 - 2,1 | M | I a | 29 |
| 2,33 | S | b | 3 |
| 3,4 - 4,2 | M | c | 2 |

IR:
1625 $cm^{-1}$ = C=N-Bande
3430 $cm^{-1}$ = NH-Bande

MS:
Molmasse = 310

Beispiel Nr. 8

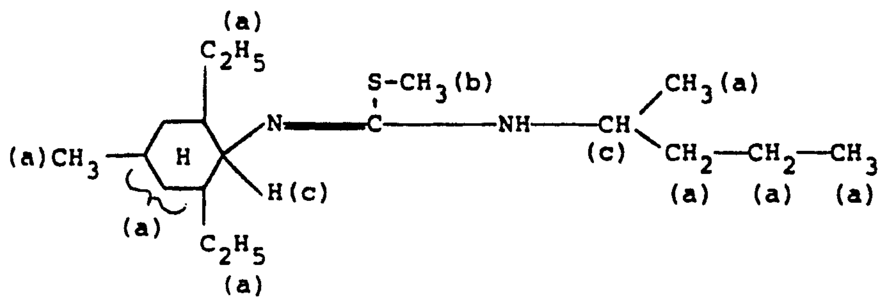

| Shift ppm | Multiplizität | Zuordnung | Intensität |
|---|---|---|---|
| NMR: | | | |
| 0,6 - 2,0 | M | I a | 31 |
| 2,32 | S | b | 3 |
| 3,3 - 4,1 | M | c | 2 |

IR:
1625 $cm^{-1}$ = C=N-Bande
3440 $cm^{-1}$ = NH-Bande

MS:
Molmasse 312

Beispiel Nr. 9

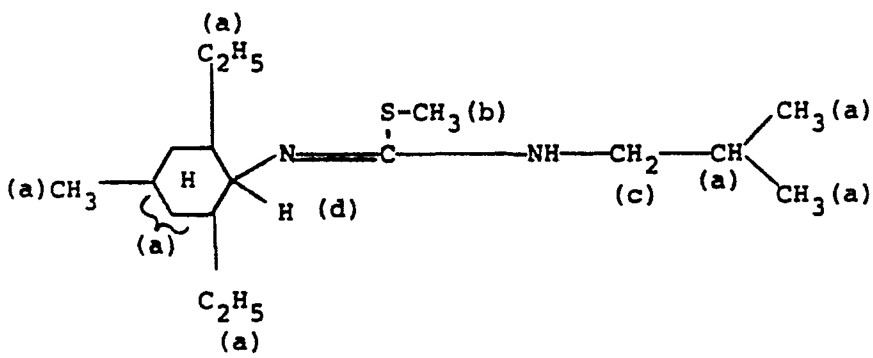

| Shift ppm | Multiplizität | Zuordnung | Intensität |
|---|---|---|---|
| NMR | | | |
| 0,7 - 2,2 | M | I a | 28 |
| 2,39 | S | b | 3 |
| 2,9 - 3,3 | M | c | 2 |
| 3,8 - 4,4 | M | d | 1 |

MS

Molmasse = 298 (Nebenkomponente = 312; ca. 5 % N,S-Dimethylderivat)

Beispiel Nr. 10

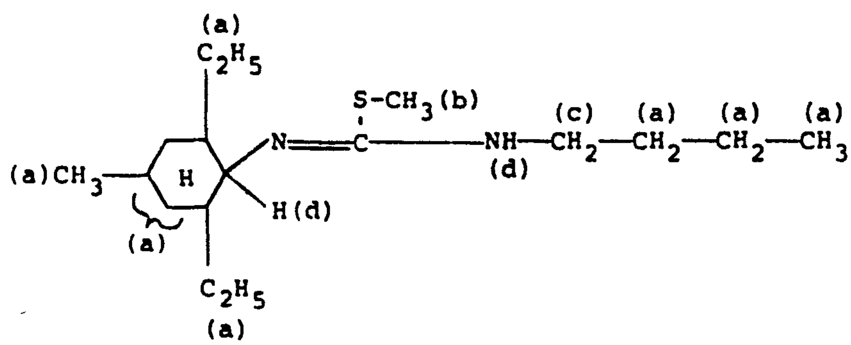

| Shift ppm | Multiplizität | Zuordnung | Intensität |
|---|---|---|---|
| NMR: | | | |
| 0,7 - 2,2 | M | I a | 27 |
| 2,39/2,41 | S/S | b | 3 |
| 3,1 - 3,5 | M | c | 2 |
| 3,6 - 4,9 | M | d | 2 |

MS:

Molmasse = 298 (Nebenkomponente = 312; ca. 5 % N,S-Dimethylderivat)

Beispiel Nr. 11

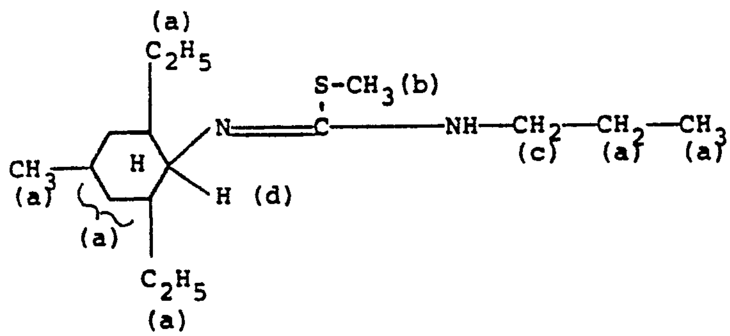

| Shift ppm | Multiplizität | Zuordnung | Intensität |
|---|---|---|---|
| NMR: | | | |
| 0,7 - 2,1 | M | I a | 26 |
| 2,38 | S | b | 3 |
| 3,0 - 3,4 | M | c | 2 |
| 3,6 - 4,3 | M | d | 1 |

MS:

Molmasse = 284 (Nebenkomponente = 298; ca. 5 % N,S-Dimethylderivat)

Beispiel Nr. 12

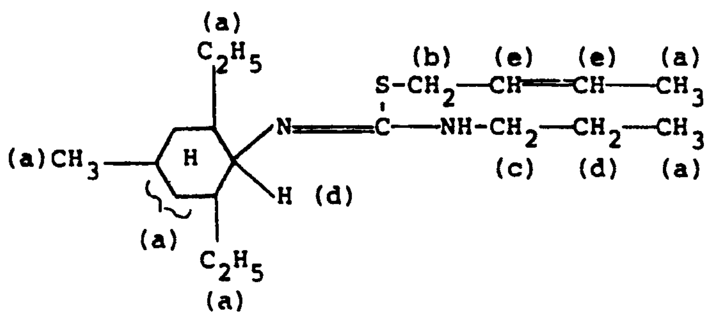

| Shift ppm | Multiplizität | Zuordnung | Intensität |
|---|---|---|---|
| NMR: | | | |
| 0,7 - 2,1 | M | I a | 28 |
| 3,18 | D | b } | 4 |
| 3,2 - 3,6 | M | c } | |
| 3,6 - 4,3 | M | d | 2 |
| 5,5 - 5,8 | M | e | ca. 2 |

MS:
Molmasse = 324

Beispiel Nr. 13

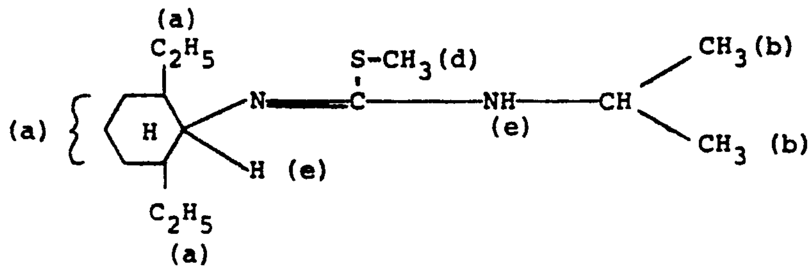

| Shift ppm | Multiplizität | Zuordnung | Intensität |
|---|---|---|---|
| 0,9 | M | I a | ~23 |
| 1,1 | D | b | |
| 1,4 | M | c | |
| 2,3 | S | d | 3 |
| 3,3 - 4,0 | breit | e | 2 |

MS: $M^+$ = 270

**Beispiel Nr. 14**

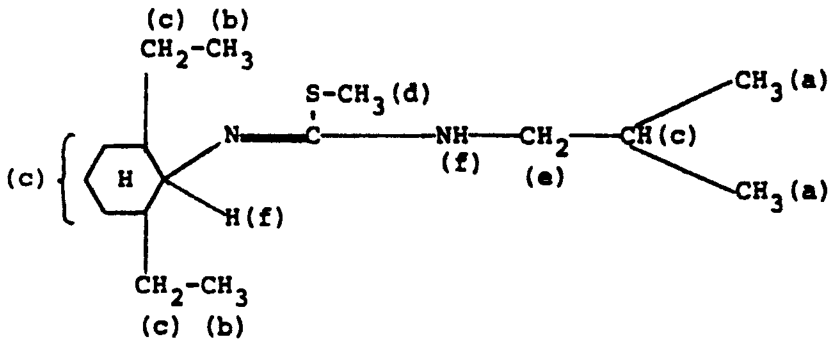

| Shift ppm | Multiplizität | Zuordnung | Intensität |
|---|---|---|---|
| 0,9 | D | I a | 10 |
| 1,0 | M | b | |
| 1,0 - 1,9 | M (breit) | c | 13 |
| 2,3 | S | d | 3 |
| 3,1 | D | e | 2 |
| 3,8 | breit | f | 1 - 2 |

MS: $M^+ = 284$

Beispiel Nr. 15

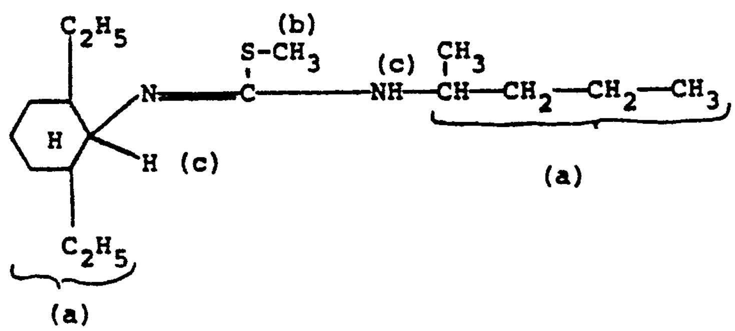

| Shift ppm | Multiplizität | Zuordnung | Intensität |
|---|---|---|---|
| 0,7 - 2,0 | M | I a | >25 |
| 2,3 | S | b | > 3 |
| 3,4 - 3,9 | M | c | 2 |

MS: $M^+$ = 298, 250 ($M^+$ - HS-$CH_3$),...

Beispiel Nr. 16

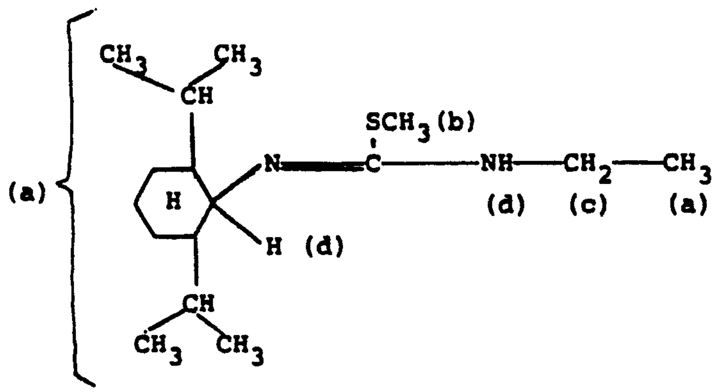

| Shift ppm | Multiplizität | Zuordnung | Intensität |
|---|---|---|---|
| NMR: | | | |
| 0,7 - 2,2 | M | I a | 25 |
| 2,39 | S | b | 3 |
| 3,28 | Q | c | 2 |
| 3,9 - 4,6 | M | d | 2 |

MS:

Molmasse = 284 (Nebenkomponente = 298; ca. 5 % S,N-Dimethyl-derivat)

Beispiel Nr. 17

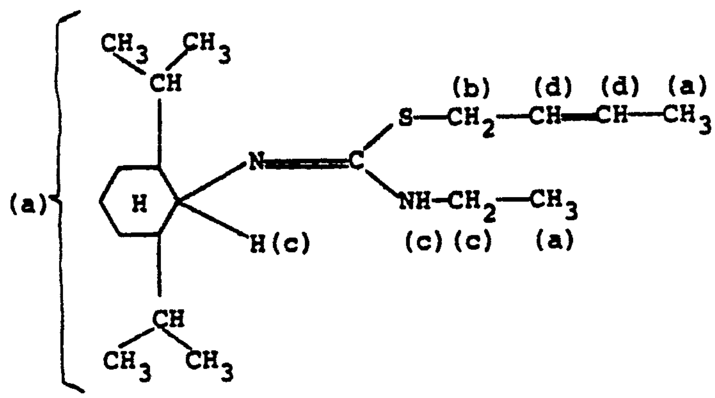

| Shift ppm | Multiplizität | Zuordnung | Intensität |
|---|---|---|---|
| NMR | | | |
| 0,7 - 2,2 | M | I a | 28 |
| 3,1 - 3,6 | M | b | 4 |
| 3,7 - 4,8 | M | c | 2 |
| 5,4 - 5,8 | M | d | 2 |

MS

Molmasse = 324

**Beispiel Nr. 18**

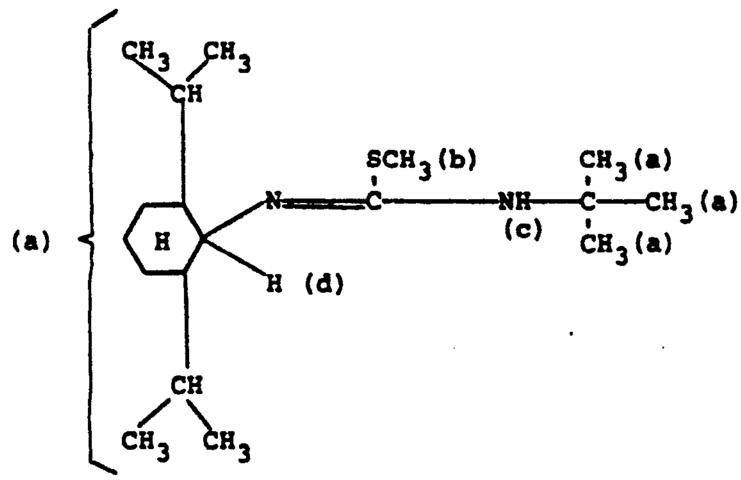

| Shift ppm | Multiplizität | Zuordnung | Intensität |
|---|---|---|---|
| 0,8 - 2,0 | M | I a | ~31 |
| 2,3 | S | b | 3 |
| 3,6 | S | c | 1 |
| 3,9 | D | d | 1 |

MS: $M^+$ = 312

Beispiel Nr. 19

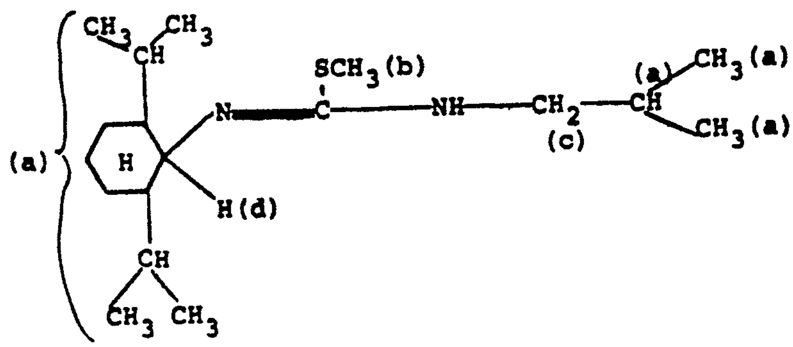

| Shift ppm | Multiplizität | Zuordnung | Intensität |
|---|---|---|---|
| **NMR** | | | |
| 0,7 - 2,2 | M | I a | 30 |
| 2,36 | S | I b | 3 |
| 2,85 - 3,2 | M | I c | 2 |
| 3,8 - 4,4 | M | I d | 1 |

MS

Molmasse 312

Beispiel Nr. 20

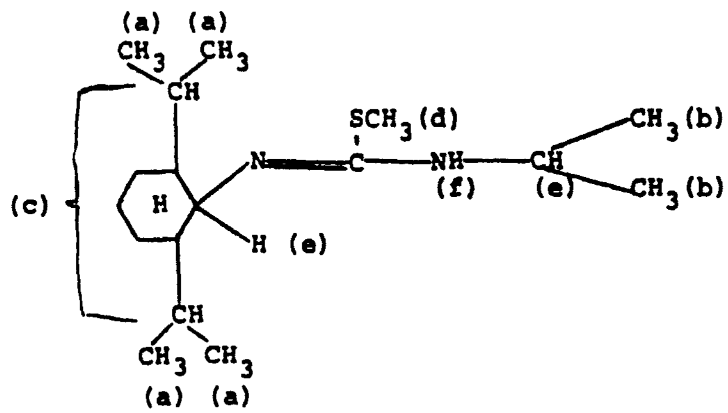

| Shift ppm | Multiplizität | Zuordnung | Intensität |
|---|---|---|---|
| 0,9 | 2 x D | I a | |
| 1,1 | 2 x D | b | ~31 |
| 1,2 - 1,9 | M | c | |
| 2,4 | 2 x D | d | 3 |
| 3,7 - 4,1 | M | e | 2 |
| 4,0 - 4,8 | breit | f | 1 ? |

MS: $M^+$ = 298, 283, ($M^+$ - $CH_3$), 255 ($M^+$ - $C_4H_7$), 250 (283-HS).

Beispiel Nr. 21

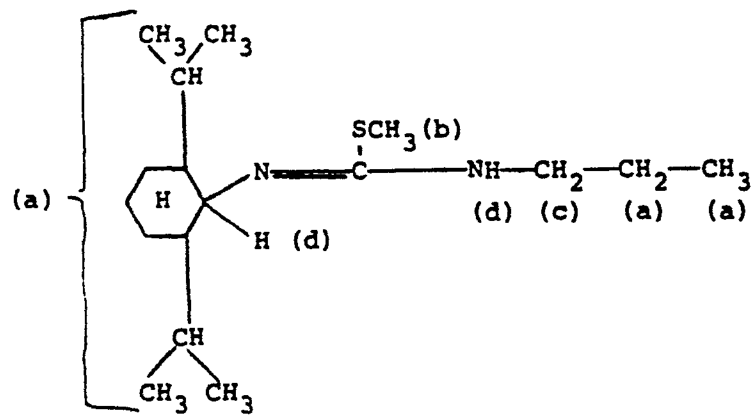

| Shift ppm | Multiplizität | Zuordnung | Intensität |
|---|---|---|---|
| 0,6 - 2,1 | M | a | 27 |
| 2,33 | S | b | 3 |
| 3,2 | T | c | 2 |
| ca. 4,05 | M | d | 2 |

MS: $M^+$ 298, 221, 207, 194, 193, 165, 139, 99, 95, 82.

Beispiel Nr. 22

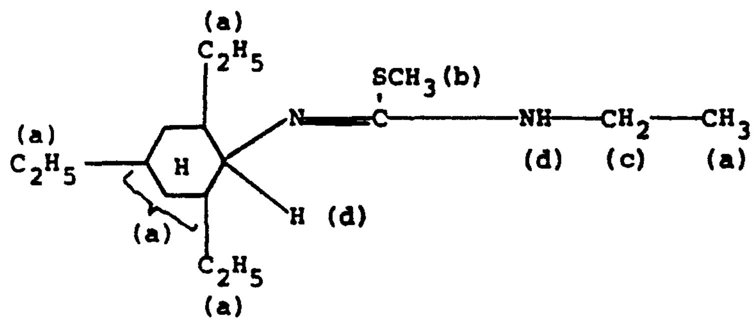

| Shift ppm | Multiplizität | Zuordnung | Intensität |
|---|---|---|---|
| NMR: | | | |
| 0,7 - 2,2 | M | I a | 25 |
| 2,37 | S (aufgesp.) | b | 3 |
| 3,1 - 3,5 | M | c | 2 |
| 3,8 - 4,4 | M | d | 2 |

MS:

Molmasse 284 (Nebenkomponente = 298; ca. 3 % = N,S-Dimethylderivat).

Beispiel Nr. 23

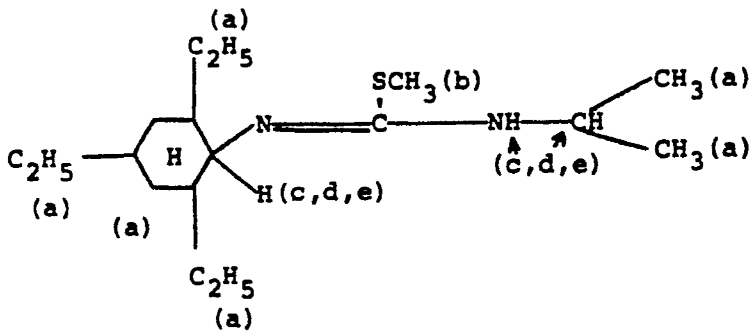

| Shift ppm | Multiplizität NMR in $CDCl_3$ | Zuordnung | Intensität |
|---|---|---|---|
| NMR: | | | |
| 0,7 - 2,0 | M | I a | 28 |
| 2,34 | S | b | 3 |
| 3,1 - 4,2 | M | c,d,e | 3 |

MS:
Molmasse = 298

Beispiel Nr. 24

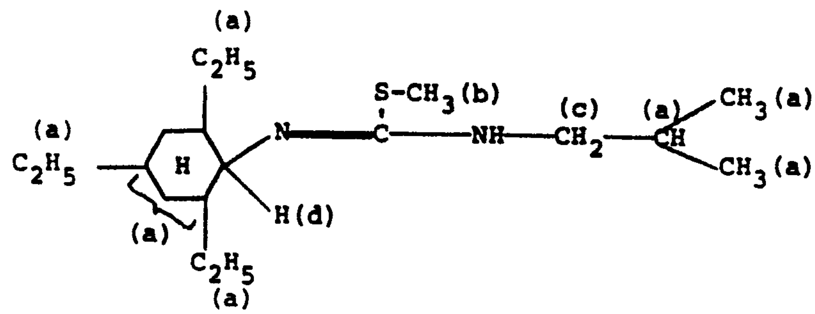

| Shift ppm | Multiplizität NMR in $CDCl_3$ | Zuordnung | Intensität |
|---|---|---|---|
| NMR: | | | |
| 0,7 - 2,2 | M | I a | 30 |
| 2,38 | S | b | 3 |
| 2,8 - 3,2 | M | c | 2 |
| 3,8 - 4,3 | M | d | 1 |

MS:
Molmasse = 312

Beispiel Nr. 25

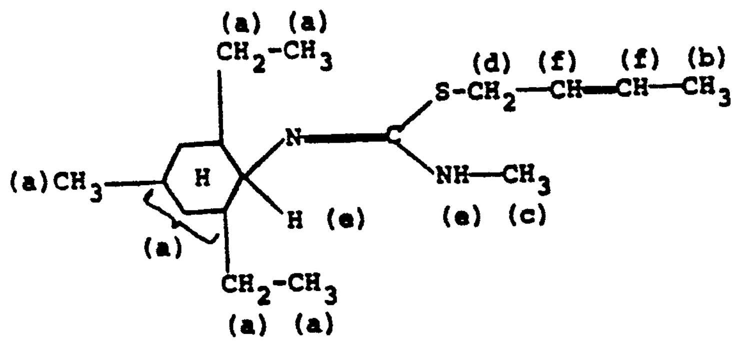

| Shift ppm | Multiplizität NMR in $CDCl_3$ | Zuordnung | | Intentsität |
|---|---|---|---|---|
| 0,5 - 2,1 | M | a | überlagert | 20 |
| 1,8 | D | b | | |
| 2,9 | M | c | | 3 |
| 3,43 | D | d | | 2 |
| 3,3 - 4,5 | M | e | | 2 |
| 5,4 - 5,9 | M | f | | 2 |

IR: 3425 (NH), 1627, 1450, 1165, 962.

MS: $M^+$ 296, 241, 208, 123, 97, 88, 83, 69.

Beispiel Nr. 26

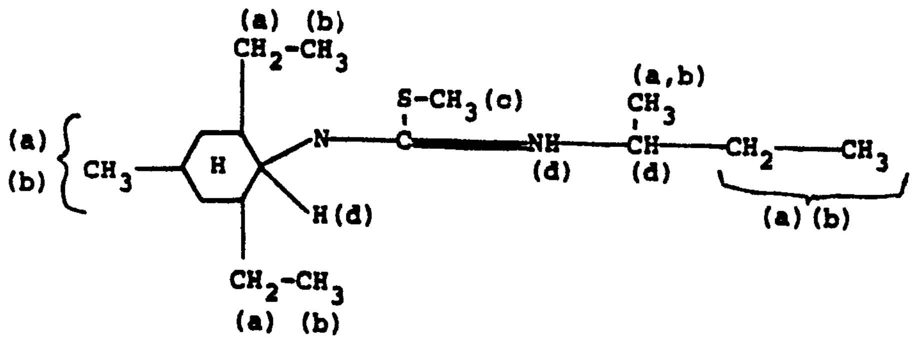

| Shift ppm | Multiplizität NMR in $CDCl_3$ | Zuordnung | Intensität |
|---|---|---|---|
| NMR: | | | |
| 0,6 - 2,0 | M | I a,b | 28 |
| 2,37 | S | c | 3 |
| 3,3 - 4,0 | M | d | 3 |

IR: 1621 $cm^{-1}$ = C=N-Bande
MS: Molmasse = 298

In Analogie zum Verfahren der Beispiele 1 b) und 3 b) wurden auch die folgenden erfindungsgemäßen Wirkstoffe hergestellt:

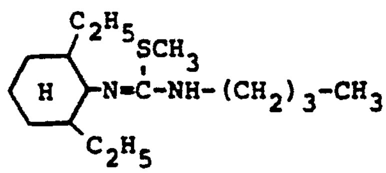

Beispiel Nr. 27

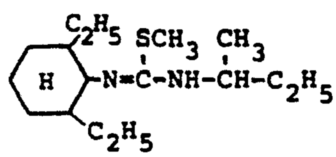

Beispiel Nr. 28

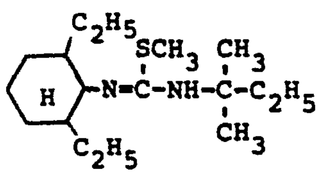

Beispiel Nr. 29

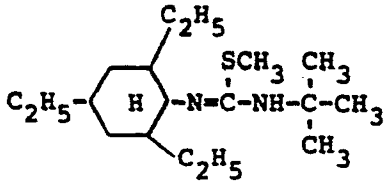

Beispiel Nr. 30

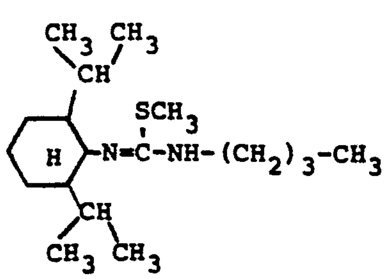

Beispiel Nr. 31

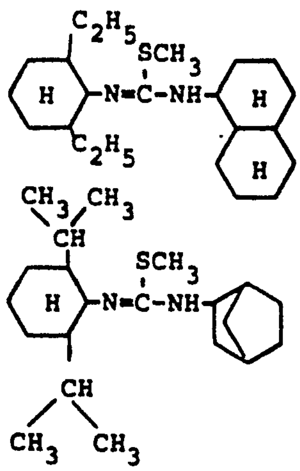

Beispiel Nr. 32

Beispiel Nr. 33

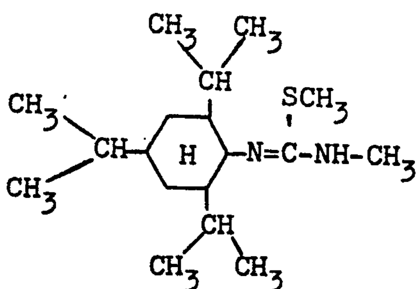

Beispiel Nr. 34

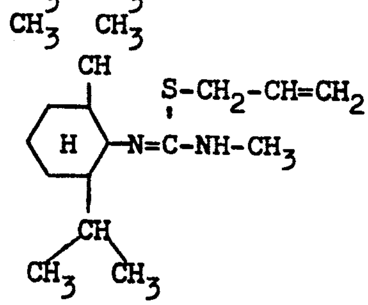

Beispiel Nr. 35

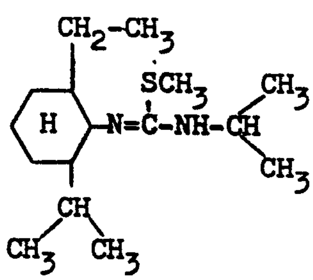

Beispiel Nr. 36

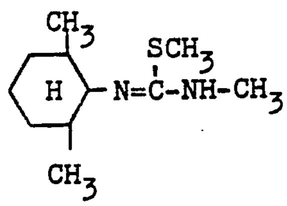

Beispiel Nr. 37

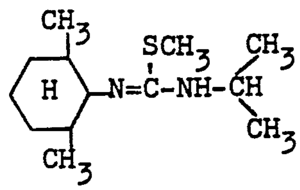

Beispiel Nr. 38

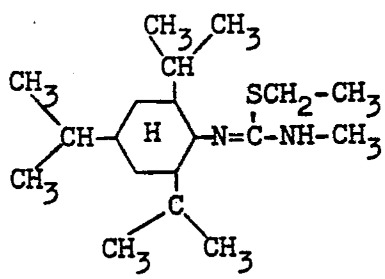

Beispiel Nr. 39

In Analogie zum Beispiel 1 a) wurden die folgenden Thioharnstoffe dargestellt, wobei unter den Formelbildern charakteristische NMR-Signale, gegebenenfalls Infrarot (IR)-Banden und gegebenenfalls Massenspektraldaten (MS) angegeben sind.

Die Multiplizität der H-NMR-Signale wird wie folgt abgekürzt:

S = Singulett
D = Dublett
T = Terzett
Q = Quartett
M = Multiplett

Bei den MS-Spektralresten ist die gefundene Molmasse bze. Massen von Molekülfragmenten angegeben.

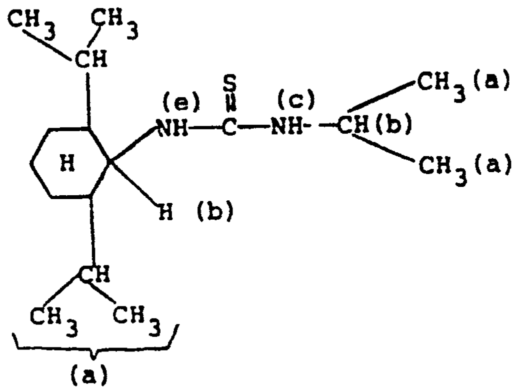

| Shift ppm | Multiplizität | Zuordnung | Intensität |
|---|---|---|---|
| **NMR** | | | |
| 0,7 - 2,0 | M | a | ca. 29 |
| 3,8 - 4,7 | M | b | ca. 2 |
| 5,8 - 6,3 | M | c | ca. 1 |

**IR**
3200 $cm^{-1}$ - 3400 $cm^{-1}$ = NH-Banden

**MS**
Molmasse = 284

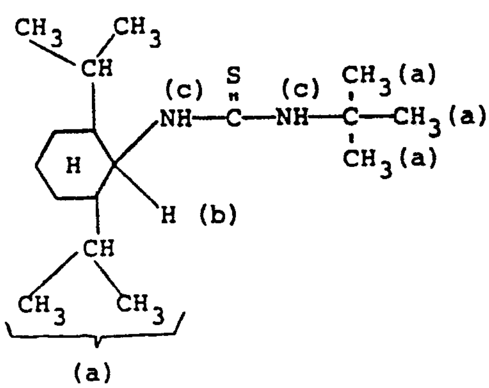

| NMR in | IR in | UV in | MS | Elementaranalyse | Sonstige |
|---|---|---|---|---|---|
| $CDCl_3$ | | | X | | |

| Shift ppm | Multiplizität | Zuordnung | Intensität |
|---|---|---|---|
| **NMR** | | | |
| 0,6 - 2,1 | M | I a | ca. 31 |
| 4,4 - 5,2 | M | b | 1 |
| 5,5 - 6,2 | M | c | 2 |

MS

Molmasse: 298

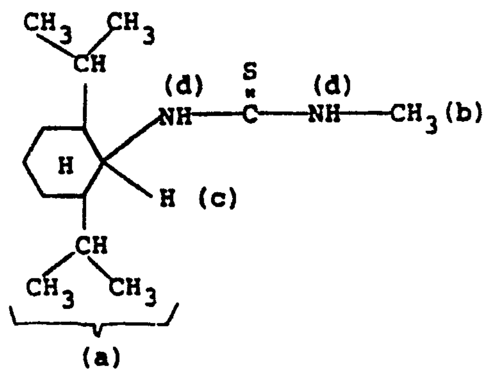

| Shift ppm | Multiplizität | Zuordnung | Intensität |
|---|---|---|---|
| NMR | | | |
| 0,7 - 2,2 | M | a | 22 |
| 3,02 | D | b | 3 |
| 4,5 - 5,0 | M | c | 1 |
| 5,8 - 6,8 | M | d | 2 |

MS

Molmasse = 256

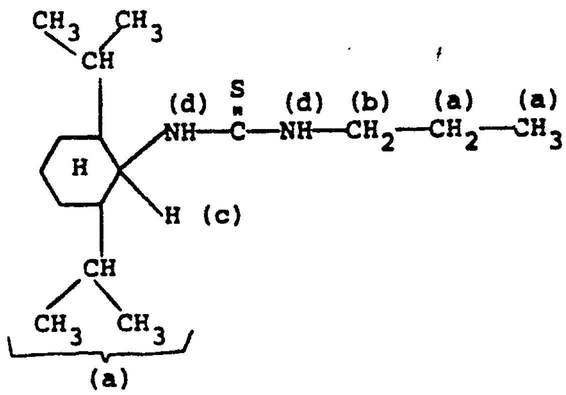

| Shift ppm | Multiplizität | Zuordnung | Intensität |
|---|---|---|---|
| 0,7 - 1,8 | M | I a | ~29 |
| 3,3 | T | b | 2 |
| 4,5 | M | c | ~ 1 |
| 5,9 | breit | d | ~ 1,5 |

MS: $M^+ = 284$

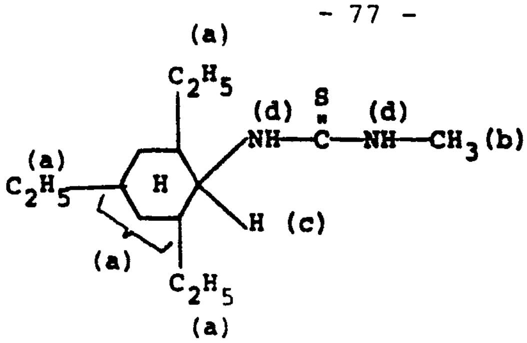

| Shift ppm | Multiplizität | Zuordnung | Intensität |
|---|---|---|---|
| **NMR** | | | |
| 0,7 - 2,2 | M | a | 22 |
| 3,04 | D | b | 3 |
| 3,9 - 4,6 | M | c | 1 |
| 5,7 - 6,8 | M | d | 2 |

**MS**

Molmasse = 256

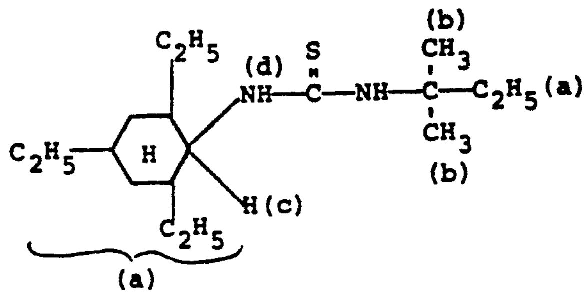

| Shift ppm | Multiplizität | Zuordnung | Intensität |
|---|---|---|---|
| 0,5 - 2,2 | M | a | überlagert |
| 1,38 | S | b | |
| ca. 3,8 - 4,6 | M (sehr breit) | c | überlagert |
| ca. 5,8 | M (breit) | d | |

MS: $M^+$ 312, 249, 147, 137, 77.

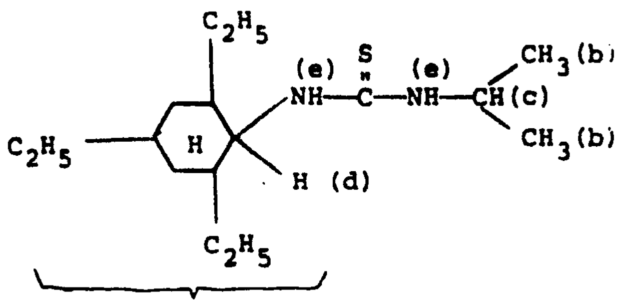

(a)

| Shift ppm | Multiplizität | Zuordnung |
|---|---|---|
| 0,5 - 2,1 | M | a |
| 1,23 | D | b |
| ca. 4,2 | M (sehr breit) | c und d |
| ca. 5,6 | M | e |

MS: $M^+$ 284, 137, 120, 85, 69.

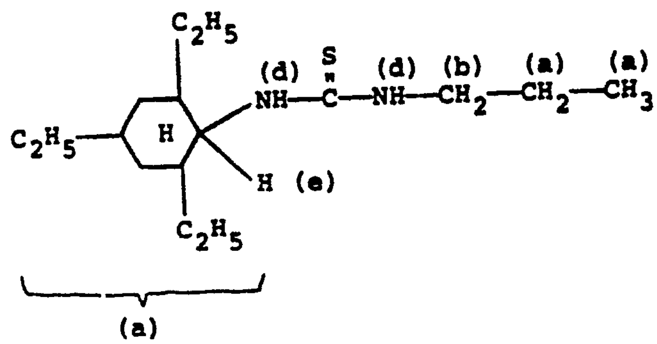

| Shift ppm | Multiplizität | Zuordnung | Intensität |
|---|---|---|---|
| 0,3 - 2,1 | M | a | 27 |
| 3,42 | Q | b | 2 |
| ca. 4,2 | M (sehr breit) | d | |
| 5,7 - 7,8 | M | c | 2 |

MS: $M^+$ 284, 180, 166, 137, 120, 95, 84, 81.

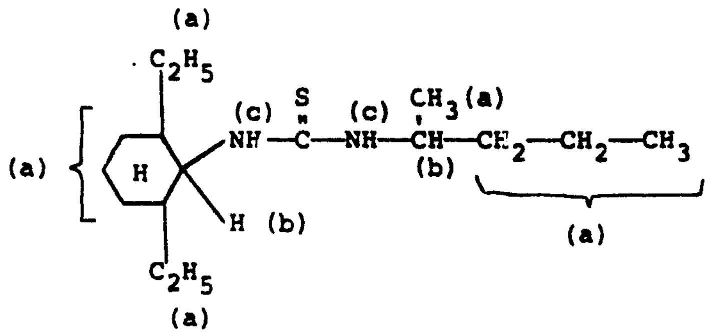

| Shift ppm | Multiplizität | Zuordnung | Intensität |
|---|---|---|---|
| **NMR** | | | |
| 0,7 - 2,1 | M | a | ca. 29 |
| 3,9 - 4,5 | M | b | 2 |
| 5,8 - 6,3 | M | c | 1 |

IR

3050 - 3400 $cm^{-1}$ = NH-Banden

MS

Molmasse = 284

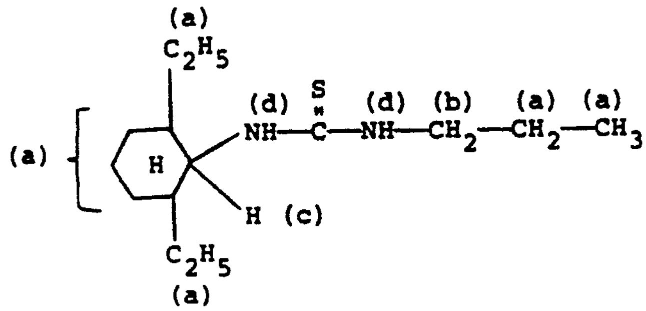

| Shift ppm | Multiplizität | Zuordnung | Intensität |
|---|---|---|---|
| 0,5 - 2,1 | M | a | 23 |
| 3,45 | Q | b | 2 |
| 3,8 - 5,0 | M | c | 1 |
| 5,8 - 6,7 | M | d | 2 |

MS: $M^+$ 256, 119, 109, 58, 55, 43, 41.

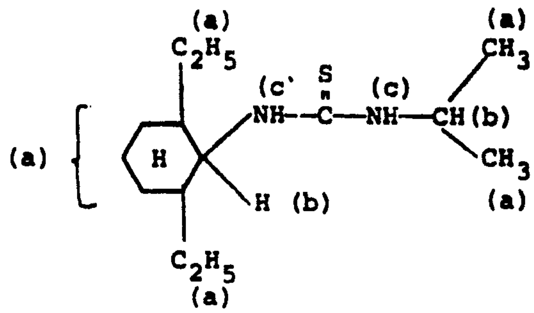

| Shift ppm | Multiplizität | Zuordnung | Intensität |
|---|---|---|---|
| 0,8 - 1,8 | M+D | I a | 24 |
| 4,3 | breit | b | 2 |
| 6,4 | breit | c | 2 |

MS: $M^+$ = 256, 223 ($M^+$ - SH).

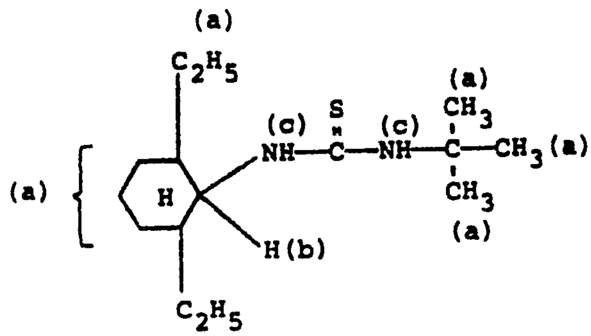

| Shift ppm | Multiplizität | Zuordnung | Intensität |
|---|---|---|---|
| **NMR** | | | |
| 0,6 - 2,1 | M | a | ca. 28 |
| 4,2 - 4,6 | M | b | ca. 1 |
| 6,1 | M | c | ca. 1 |

**IR**
3100 - 3400 $cm^{-1}$ = NH-Banden

**MS**
Molmasse 270

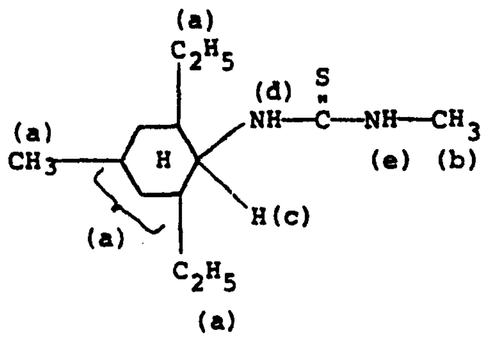

| Shift ppm | Multiplizität | Zuordnung | Intensität |
|---|---|---|---|
| 0,4 - 2,9 | M | a | 20 |
| 2,98 u. 3,05 | S'S | b | 3 |
| ca. 4,4 | M | c | 1 |
| ca. 6,0 | M | d oder e | 1 |
| ca. 6,6 | M | e oder d | 1 |

IR: 3270 u. 3055 (NH), 2920, 1540, 1343, 730 $cm^{-1}$
MS: $M^+$ 242, 123, 97, 91, 83, 81, 74, 69.

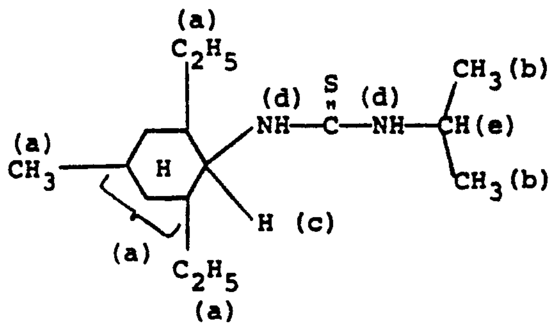

| Shift ppm | Multiplizität | Zuordnung | Intensität |
|---|---|---|---|
| 0,3 - 2,8 | M | a | überlagert 26 |
| 1,25 | D | b | |
| 3,7 - 4,75 | M | c | 2 |
| 5,5 - 6,4 | M | d | 2 (DCl-austauschbar) |

IR: 3260 u. 3055 (NH), 2955, 1525, 1343, 721 $cm^{-1}$.

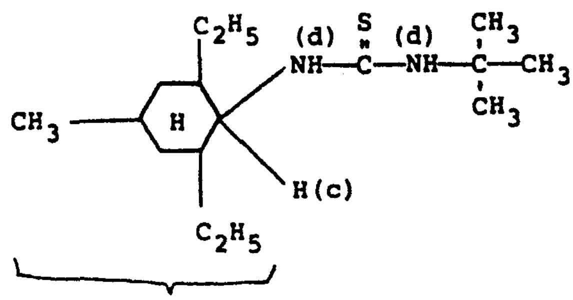

(b)

(a)

| Shift ppm | Multiplizität | Zuordnung |
|---|---|---|
| **1-H-NMR:** | | |
| 0,5 - 2,2 | M | a |
| 1,45 | S | b |
| ca. 4,8 | M | c |
| 5,5 - 6,3 | M | d |

**13-C-NMR:**

12,6 ($-CH_3$ in $-CH_2CH_3$)

29,7 ($-CH_3$ in $-C(CH_3)_3$

180,0/181,3 (C=S)

22,0 - 63,3 (alle anderen C)

IR: 3290 (NH), 1520, 1315, 1201, 698 $cm^{-1}$.

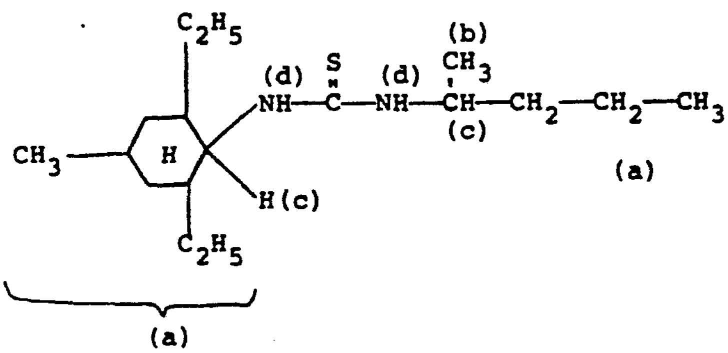

| Shift ppm | Multiplizität | Zuordnung | | Intensität |
|---|---|---|---|---|
| 0,4 - 2,7 | M | a | überlagert | 30 |
| 1,2 | D | b | | |
| 3,6 - 4,6 | M | c | | 2 |
| 5,5 - 6,2 | M | d | | 2 |

IR: 3260 u. 3050 (NH), 2945, 1528 $cm^{-1}$.

Herstellung von Vorstufen für Ausgangsverbindungen

Beispiel A 1

4-Cyclohexyl-2,6-diäthyl-anilin:

300 g 4-Amino-cyclohexylbenzol, 5,0 g Aluminiumgranulat und 17 g wasserfreies Aluminiumchlorid werden in einem Stahlautoklaven auf 250°C erhitzt und Äthylen bis zu einem Innendruck von 200 atü aufgepreßt. Nach Druckabfall wird weiter Äthylen zugepumpt, bis die Aufnahme beendet ist; Dauer ca. 7 Stunden. Nach dem Erkalten wird der Ansatz mit 500 ml Benzol, 300 ml 40 %iger Natronlauge und 500 ml Wasser 15 Minuten bei 40 - 50°C verrührt, die Benzolphase abgetrennt, mit Wasser gewaschen, über Kaliumcarbonat getrocknet und fraktioniert.
Kp 148 - 150°C/0,8 Torr; Ausbeute 318 g.

In analoger Weise können die folgenden Anilinderivate hergestellt werden:

2,4,6-Triäthyl-anilin; Kp 89-91°C/0,6 Torr
4-n-Propyl-2,6-diäthyl-anilin; Kp 102°C/1,4 Torr
4-Isopropyl-2,6-diäthyl-anilin; Kp 103-105°C/2,0 Torr
4-n-Butyl-2,6-diäthyl-anilin; Kp 117-118°C/2,0 Torr
4-Isobutyl-2,6-diäthyl-anilin; Kp 97-99°C/0,7 Torr
4-tert.-Butyl-2,6-diäthyl-anilin; Kp 89-91°C/0,6 Torr
4-sek.-Butyl-2,6-diäthyl-anilin; Kp 108-113°C/1,7 Torr
3,4-Dimethyl-2,6-diäthyl-anilin; Kp 147-148°C/15 Torr
3,5-Dimethyl-2,6-diäthyl-anilin; Kp 146-154°C/15 Torr F: 47-50°C

2-Äthyl-6-isopropyl-anilin; Kp 127-128°C/16 Torr
2-Äthyl-6-sek.-butyl-anilin; Kp 136-140°C/20 Torr
4-[Pentyl-(3)]-2,6-diäthyl-anilin; Kp 106-107°C/1,2 Torr
4-n-Hexyl-2,6-diäthyl-anilin; Kp 120-123°C/0,5 Torr

**Beispiel A 2**

2,6-Bis-(pentyl-(2))-anilin:

170 g Anilin, 5 g Aluminiumgranulat und 15 g wasserfreies Aluminiumchlorid werden in einem Stahlautoklaven auf 300°C erhitzt und 300 g Penten-(1) innerhalb von etwa 5 Stunden bis zu einem Innendruck von 300 atü eingepumpt. Anschließend wird der Ansatz noch 6 Stunden bei 300°C gehalten, wobei der Innendruck auf 107 atü fällt. Nach dem Erkalten wird der Autoklaveninhalt mit 500 ml Benzol, 250 ml 40%iger Natronlauge und 300 ml Wasser 15 Minuten bei 30 - 40°C verrührt, die Benzolschicht mit Wasser gewaschen, über Kaliumcarbonat getrocknet und fraktioniert. Man erhält 113 g 2-Mono-pentyl-(2)-anilin, Kp 78-82°C/0,6 Torr und 131 g 2,6-Bis-pentyl-(2)-anilin, Kp 168 - 174°C/1,5 Torr.

Analog erhält man aus Anilin und Cyclopenten
2-Cyclopentylanilin; Kp 102-109°C/1,7 Torr und
2,6-Di-cyclopentyl-anilin; Kp 159-165°C/1,5 Torr;
aus p-Toluidin und Cyclopenten
4-Methyl-2-cyclopentyl-anilin; Kp 104-106°C/0,7 Torr und
4-Methyl-2,6-dicyclopentyl-anilin; Kp 157-158°C/0,8 Torr;
aus p-Toluidin und Buten-(1)
4-Methyl-2-sek.-butyl-anilin; Kp [illegible]2°C/1,0 Torr und

4-Methyl-2,6-di-sek.-butyl-anilin; Kp 121-123°C/3,0 Torr. Aus o-Toluidin und Isobuten erhält man unter Verwendung eines geeigneten Katalysators bei 200°C das 6-Methyl-2,4-di-tert.-butyl-anilin; Kp 101-103°C/1,1 Torr.

Beispiel A 3

2,6-Diäthyl-cyclohexylamin:

500 g 2,6-Diäthyl-anilin und 500 ml Tetrahydrofuran werden mit 60 g eines 5 %igen Ruthenium-Kohlekontaktes versetzt und im Hochdruck-Rührautoklaven bei 120 - 150° und 200 atü bis zur beendeten Wasserstoffaufnahme hydriert. Danach wird vom Katalysator abfiltriert und das Filtrat fraktioniert.
Kp 55 - 57°C/0,6 Torr, Ausbeute 475 g.

Im Gaschromatogramm erscheinen 3 Komponenten im Verhältnis 48%:12%:40%.

Analog können erhalten werden
aus 2,6-Dimethylanilin das
2,6-Dimethyl-cyclohexylamin Kp 62-64°C/24 Torr.
Im Gaschromatogramm erscheinen 2 Komponenten im Verhältnis 60%:40%.

aus 2-Methyl-4-äthyl-anilin das
2-Methyl-2-äthyl-cyclohexylamin Kp 97 - 81°C/24 Torr
Im Gaschromatogramm 3 Komponenten 58 %:6 %:25 %

aus 2-Äthyl-6-isopropyl-anilin das
2-Äthyl-6-isopropyl-cyclohexylamin Kp 59-62°C/1,1 Torr
Im Gaschromatogramm 3 Komponenten 50 %:20 %:19 %
neben 2 kleineren Komponenten.

aus 2,6-Diisopropylamin das
2,6-Diisopropyl-cyclohexylamin Kp 74°C/0,5 Torr
Im Gaschromatogramm 3 Komponenten 3 %:29 %:66 %.

aus 3-Methyl-2,6-diäthylanilin das
3-Methyl-2,6-diäthyl-cyclohexylamin Kp 51-54°C/0,9 Torr
Im Gaschromatogramm 3 Komponenten 8 %:37 %:53 %

aus 4-Methyl-2,6-diäthylanilin das
4-Methyl-2,6-diäthyl-cyclohexylamin Kp 99-101°C/12 Torr
Im Gaschromatogramm 3 Komponenten 78 %:20 %:1 %.

aus 2,4,6-Triäthylanilin das
2,4,6-Triäthyl-cyclohexylamin; Kp 76-78°C/0,7 Torr
Im Gaschromatogramm 2 Komponenten 84 %:16 %.

aus 4-tert.-Butyl-2,6-diäthyl-anilin das
4-tert.-Butyl-2,6-diäthyl-cyclohexylamin; Kp 89-91°C/1,6 Torr
Im Gaschromatogramm 5 Komponenten 31 %:9 %:24 %:1 %: 31 %

aus 4-Cyclohexyl-2,6-diäthyl-anilin das
4-Cyclohexyl-2,6-diäthyl-cyclohexylamin Kp 126-131°C/1,7 Torr
Im Gaschromatogramm 5 Komponenten 5 %:69 %:2 %:5 %:8 %
sowie weitere Komponenten in geringeren Anteilen.

Die Konstitutionen der vorstehenden Verbindungen wurden durch Elementaranalyse, H-NMR- und Massenspektrum gesichert.

**Beispiel A 4**

**2,6-Diäthyl-cyclohexyl-isothiocyanat**

200 g 2,6-Diäthylcyclohexylanilin in 300 ml Methylenchlorid werden zu einem Gemisch von 500 ml Methylenchlorid, 500 ml Wasser, 200 g Calciumcarbonat und 178 g Thiophosgen zugetropft. Man erwärmt zum Rückfluß bis die Gasentwicklung beendet ist. Danach wird von Feststoffen abfiltriert, die Methylenchloridphase über Calciumchlorid getrocknet und fraktioniert.
Kp 110-112°C/1,5 Torr; Ausbeute 215 g.

Analog erhält man aus den entsprechenden Cyclohexylaminderivaten die folgenden Isothiocyanate

2,6-Dimethyl-cyclohexylisothiocyanat; Kp 121-122°C/11 Torr
2-Methyl-6-äthyl-cyclohexylisothiocyanat; Kp 92-95°C/0,8 Torr
2-Äthyl-6-isopropyl-cyclohexylisothiocanat; Kp 111-113°C/0,8 Torr
2,6-Diisopropyl-cyclohexylisothiocyanat; Kp 119-121°C/1,3 Torr
3-Methyl-2,6-diäthyl-cyclohexylisothiocyanat; Kp 101-103°C/1,0 Torr
4-Methyl-2,6-diäthyl-cyclohexylisothiocyanat; Kp 110-112°C/1,3 Torr
2,4,6-Triäthyl-cyclohexylisothiocyanat; Kp 120-122°C/1,5 Torr

4-tert-Butyl-2,6-diäthyl-cyclohexylisothiocyanat; Kp 130-132°C/ 1,0 Torr

4-Cyclohexyl-2,6-diäthyl-cyclohexylisothiocyanat; Kp 164-166°C/ 1,0 Torr.

Patentansprüche

1. Substituierte N-Cyclohexyl-isothioharnstoffe der Formel

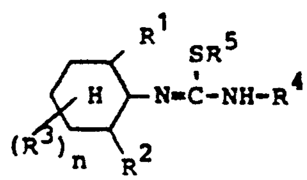

(I)

in welcher

$R^1$ und $R^2$ gleich oder verschieden sein können und für Alkyl oder für gegebenenfalls durch Alkyl substituiertes Cycloalkyl stehen,

$R^3$ für Alkyl oder für gegebenenfalls durch Alkyl substituiertes Cyclalkyl steht,

n für eine ganze Zahl von 0 bis 2,

$R^4$ für Alkyl, Alkenyl, Cycloalkenyl oder für gegebenenfalls durch Alkyl, $CF_3$ oder Cycloalkyl substituiertes Cycloalkyl

und $R^5$ für Alkyl, Alkenyl oder Cycloalkyl steht.

2. Substituierte N-Cyclohexyl-isothioharnstoffe gemäß Anspruch 1, gekennzeichnet durch die Formel

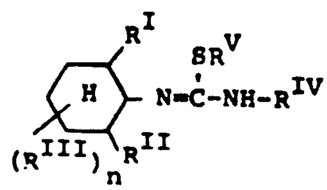

(Ia)

in welcher

$R^{I}$ für Alkyl ($C_1$-$C_6$) oder Cycloalkyl ($C_5$-$C_6$),

$R^{II}$ für Alkyl ($C_1$-$C_6$) oder Cycloalkyl ($C_5$-$C_6$),

$R^{III}$ für Alkyl ($C_1$-$C_6$) oder Cycloalkyl ($C_5$-$C_6$),

n für eine ganze Zahl von O bis 2,

$R^{IV}$ für Alkyl ($C_1$-$C_{12}$), für gegebenenfalls durch Alkyl ($C_1$-$C_4$), $CF_3$ oder Cycloalkyl ($C_3$-$C_6$) substituiertes Cycloalkyl ($C_3$-$C_{12}$),

für Alkenyl ($C_3$-$C_{12}$)

oder für Cycloalkenyl ($C_5$-$C_6$)

und $R^{V}$ für Alkyl ($C_1$-$C_6$), Alkenyl ($C_3$-$C_6$) oder für Cycloalkyl ($C_3$-$C_6$) steht.

3. Substituierter N-Cyclohexyl-isothioharnstoff gemäß Anspruch 1, gekennzeichnet durch die Formel

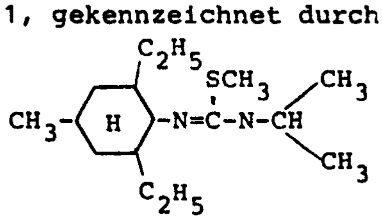

4. Substituierter N-Cyclohexyl-isothioharnstoff gemäß Anspruch 1, gekennzeichnet durch die Formel

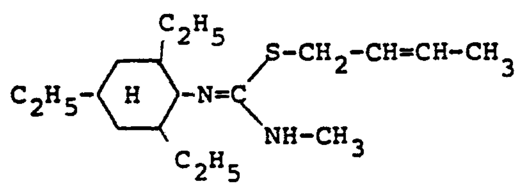

5. Substituierter N-Cyclohexyl-isothioharnstoff gemäß Anspruch 1, gekennzeichnet durch die Formel

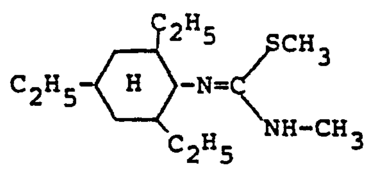

6. Substituierter N-Cyclohexyl-isothioharnstoff gemäß Anspruch 1, gekennzeichnet durch die Formel

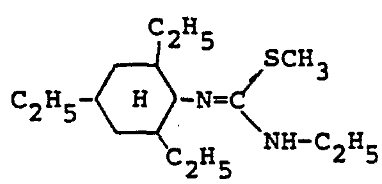

7. Substituierter N-Cyclohexyl-isothioharnstoff gemäß Anspruch 1, gekennzeichnet durch die Formel

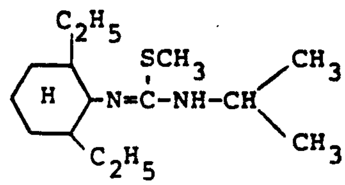

8. Substituierter N-Cyclohexyl-isothioharnstoff gemäß Anspruch 1, gekennzeichnet durch die Formel

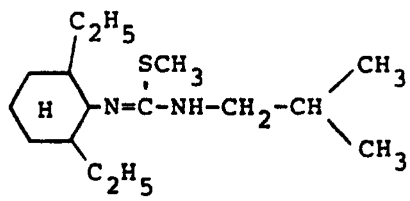

9. Substituierter N-Cyclohexyl-isothioharnstoff gemäß Anspruch 1, gekennzeichnet durch die Formel

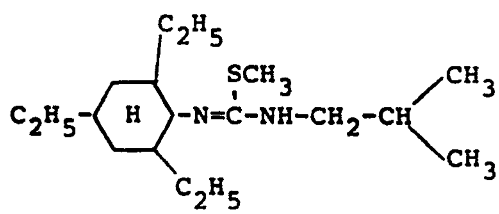

10. Substituierter N-Cyclohexyl-isothioharnstoff gemäß Anspruch 1, gekennzeichnet durch die Formel

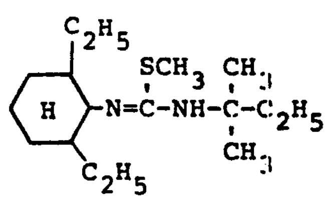

11. Verfahren zur Herstellung von substituierten N-Cyclohexyl-isothioharnstoffen der Formel

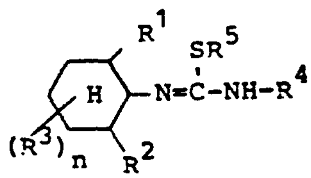

(I)

in welcher

$R^1$ und $R^2$ gleich oder verschieden sein können und für Alkyl oder für gegebenenfalls durch Alkyl substituiertes Cycloalkyl stehen,

$R^3$ für Alkyl oder für gegebenenfalls durch Alkyl substituiertes Cycloalkyl steht,

n für eine ganze Zahl von 0 bis 2,

$R^4$ für Alkyl, Alkenyl, Cycloalkenyl oder für gegebenenfalls durch Alkyl, $CF_3$ oder Cycloalkyl substituiertes Cycloalkyl

und $R^5$ für Alkyl, Alkenyl oder Cycloalkyl steht,

dadurch gekennzeichnet, daß man N-Cyclohexylthioharnstoffe der Formel

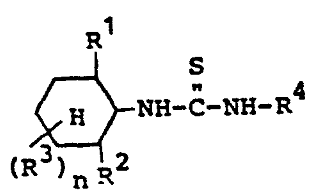

(II)

in welcher $R^1$, $R^2$, $R^3$, $R^4$ und n die oben angegebene Bedeutung besitzen,

mit einer Verbindung der allgemeinen Formel

$$R^5\text{-X} \qquad \text{(III)}$$

in welcher $R^5$ die oben angegebene Bedeutung besitzt und X für einen anionisch abspaltbaren Substituenten steht,

gegebenenfalls in Gegenwart eines Lösungsmittels bei Temperaturen zwischen ca. 10 und ca. 120°C umsetzt,

und die entstandenen Reaktionsprodukte nach an sich bekannten Methoden isoliert.

12. Mittel gegen tierische und pflanzliche Schädlinge, gekennzeichnet durch einen Gehalt an substituierten N-Cyclohexyl-isothioharnstoffen gemäß den Ansprüchen 1 bis 10.

13. Ektoparasititzide Mittel, gekennzeichnet durhc einen Gehalt an substituierten N-Cyclohexyl-isothioharnstoffen gemäß den Ansprüchen 1 bis 10.

14. Gegen Insekten und Akariziden wirksame Mittel, gekennzeichnet durch einen Gehalt an substituierten N-Cyclohexylisothioharnstoffen gemäß den Ansprüchen 1 bis 10.

15. Verfahren zur Herstellung von gegeben pflanzliche und tierische Schädlinge wirksamen Mitteln, dadurch gekennzeichnet, daß man substituierte N-Cyclohexylisothioharnstoffe gemäß den Ansprüchen 1 bis 10 mit inerten, nichttoxischen Verdünnungsmitteln und/oder Trägerstoffen vermischt.

16. Verfahren zur Bekämpfung von Ektoparasiten, dadurch gekennzeichent, daß man von Ektoparasiten befallene Tiere mit substituierten N-Cyclohexyl-isothioharnstoffen gemäß Anspruch 1 behandelt.

Europäisches Patentamt

# EUROPÄISCHER RECHERCHENBERICHT

0003101

Nummer der Anmeldung

EP 79 10 0007

| EINSCHLÄGIGE DOKUMENTE | | | KLASSIFIKATION DER ANMELDUNG (Int.Cl.²) |
|---|---|---|---|
| Kategorie | Kennzeichnung des Dokuments mit Angabe, soweit erforderlich, der maßgeblichen Teile | betrifft Anspruch | |
| | US - A - 4 062 892 (FANCHER UND FREIBERG)<br>* Zusammenfassung *<br>--- | 1 | C 07 C 157/14<br>A 01 N 9/12 |

RECHERCHIERTE SACHGEBIETE (Int. Cl.²)

C 07 C 157/14

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 09-04-1979 | GAUTIER |